# FASCICULE DE BREVET EUROPEEN

(11) **EP 2 531 213 B1**
(45) Date de publication et mention de la délivrance du brevet: **10.04.2019**
(21) Numéro de dépôt: 11707480.7
(22) Date de dépôt: 01.02.2011
(51) Int. Cl.: A61K 39/385, C07K 16/28, C07K 16/42, C07K 14/16, C12N 15/63, A61P 35/00, A61P 31/00

(54) **COMPLEXE MOLECULAIRE DE CIBLAGE DES ANTIGÈNES VERS LES CELLULES PRÉSENTATRICE D'ANTIGÈNE ET SES APPLICATIONS POUR LA VACCINATION**
MOLEKULARER KOMPLEX ZUR RICHTUNG VON ANTIGENEN GEGEN ZELLEN MIT ANTIGENEN UND SEINE VERWENDUNG FÜR IMPFSTOFFE
MOLECULAR COMPLEX FOR TARGETING ANTIGENS TOWARDS CELLS COMPRISING ANTIGENS AND USES THEREOF FOR VACCINATION

(30) Priorité: 01.02.2010 FR 1000392
(43) Date de publication de la demande: 12.12.2012
(73) Titulaire: Commissariat à l'Énergie Atomique et aux Énergies Alternatives, 75015 Paris (FR)
(72) Inventeur: LEONETTI, Michel, 91620 Nozay (FR); SAVATIER, Alexandra, 91150 Etampes (FR); GADZINSKI, Adeline, 77270 Villeparisis (FR); BOULAIN, Jean-Claude, 91140 Villlebon (FR)
(74) Mandataire: Cabinet Plasseraud
(86) Numéro de dépôt international: PCT/IB2011/050437
(87) Numéro de publication internationale: WO 2011/092675

(56) Documents cités:
- WO-A1-98/34956
- WO-A2-03/084467
- WO-A2-2005/097179
- WO-A2-2007/015704
- US-A1- 2004 258 688
- YANG LILI ET AL: "Engineered lentivector targeting of dendritic cells for in vivo immunization.", NATURE BIOTECHNOLOGY MAR 2008 LNKD- PUBMED:18297056, vol. 26, no. 3, mars 2008 (2008-03), pages 326-334, XP002598156, ISSN: 1546-1696 cité dans la demande
- WANG SUNA ET AL: "HBcAg18-27 epitope fused to HIV-Tat 49-57 adjuvanted with CpG ODN induces immunotherapeutic effects in transgenic mice.", IMMUNOLOGY LETTERS 4 JAN 2010 LNKD- PUBMED:19883689, vol. 127, no. 2, 4 janvier 2010 (2010-01-04), pages 143-149, XP002598157, ISSN: 1879-0542 cité dans la demande
- GADZINSKI A ET AL: "P02-01. Elicitation of a humoral immune response towards non-immunogenic peptides using the transcriptional transactivator of HIV-1", RETROVIROLOGY, BIOMED CENTRAL LTD., LONDON, GB LNKD- DOI:10.1186/1742-4690-6-S3-P6, vol. 6, no. Suppl 3, 22 octobre 2009 (2009-10-22), page P6, XP021064175, ISSN: 1742-4690
- TURBANT S ET AL: "Cynomolgus macaques immunized with two HIV-1 Tat stabilized proteins raise strong and long-lasting immune responses with a pattern of Th1/Th2 response differing from that in mice", VACCINE, ELSEVIER LTD, GB LNKD- DOI:10.1016/J.VACCINE.2009.06.083, vol. 27, no. 39, 27 août 2009 (2009-08-27) , pages 5349-5356, XP026614434, ISSN: 0264-410X [extrait le 2009-07-14] cité dans la demande
- LECOQ A ET AL: "Increasing the humoral immunogenic properties of the HIV-1 Tat protein using a ligand-stabilizing strategy", VACCINE, ELSEVIER LTD, GB LNKD- DOI:10.1016/J.VACCINE.2008.02.057, vol. 26, no. 21, 19 mai 2008 (2008-05-19), pages 2615-2626, XP022639506, ISSN: 0264-410X [extrait le 2008-03-18] cité dans la demande
- KITTIWORAKARN JONGRAK ET AL: "HIV-1 Tat raises an adjuvant-free humoral immune response controlled by its core region and its ability to form cysteine-mediated oligomers.", THE JOURNAL OF BIOLOGICAL CHEMISTRY 10 FEB 2006 LNKD- PUBMED:16321975, vol. 281, no. 6, 10 février 2006 (2006-02-10), pages 3105-3115, XP002598158, ISSN: 0021-9258 cité dans la demande
- WATSON K ET AL: "Interaction of the transactivating protein HIV-1 tat with sulphated polysaccharides", BIOCHEMICAL PHARMACOLOGY, PERGAMON, OXFORD, GB LNKD- DOI:10.1016/S0006-2952(98)00352-9, vol. 57, no. 7, 1 avril 1999 (1999-04-01), pages 775-783, XP002346129, ISSN: 0006-2952
- LEONETTI M ET AL: "Presentation of antigen in immune complexes is boosted by soluble bacterial immunoglobulin binding proteins", THE JOURNAL OF EXPERIMENTAL MEDICINE, ROCKEFELLER UNIVERSITY PRESS, US LNKD- DOI:10.1084/JEM.189.8.1217, vol. 189, no. 8, 19 avril 1999 (1999-04-19), pages 1217-1228, XP002442500, ISSN: 0022-1007 cité dans la demande
- LÉONETTI M ET AL: "Increasing immunogenicity of antigens fused to Ig-binding proteins by cell surface targeting.", JOURNAL OF IMMUNOLOGY (BALTIMORE, MD. : 1950) 15 APR 1998 LNKD- PUBMED:9558086, vol. 160, no. 8, 15 avril 1998 (1998-04-15), pages 3820-3827, XP002598159, ISSN: 0022-1767 cité dans la demande
- MITSUI HIROSHI ET AL: "Polyarginine-mediated protein delivery to dendritic cells presents antigen more efficiently onto MHC class I and class II and elicits superior antitumor immunity", JOURNAL OF INVESTIGATIVE DERMATOLOGY, NATURE PUBLISHING GROUP, GB LNKD- DOI:10.1038/SJ.JID.5700335, vol. 126, no. 8, 1 août 2006 (2006-08-01), pages 1804-1812, XP002567496, ISSN: 0022-202X [extrait le 2006-04-27] cité dans la demande
- BOZZACCO LEONIA ET AL: "DEC-205 receptor on dendritic cells mediates presentation of HIV gag protein to CD8+ T cells in a spectrum of human MHC I haplotypes.", PROCEEDINGS OF THE NATIONAL ACADEMY OF SCIENCES OF THE UNITED STATES OF AMERICA 23 JAN 2007 LNKD- PUBMED:17229838, vol. 104, no. 4, 23 janvier 2007 (2007-01-23), pages 1289-1294, XP002598160, ISSN: 0027-8424 cité dans la demande
- KRISHNAMACHARI YOGITA ET AL: "Innovative strategies for co-delivering antigens and CpG oligonucleotides.", ADVANCED DRUG DELIVERY REVIEWS 28 MAR 2009 LNKD- PUBMED:19272328, vol. 61, no. 3, 28 mars 2009 (2009-03-28), pages 205-217, XP002598161, ISSN: 1872-8294
- DESPINA RUDOLF ET AL: "Potent costimulation of human CD8 T cells by anti-4-1BB and anti-CD28 on synthetic artificial antigen presenting cells", CANCER IMMUNOLOGY, IMMUNOTHERAPY, SPRINGER, BERLIN, DE LNKD- DOI:10.1007/S00262-007-0360-X, vol. 57, no. 2, 27 juillet 2007 (2007-07-27), pages 175-183, XP019561064, ISSN: 1432-0851

## Description

La présente invention concerne un nouveau complexe moléculaire de ciblage des antigènes vers les cellules présentatrices d'antigène comprenant au moins un antigène associé à au moins deux ligands de molécules exprimées à la surface des cellules présentatrices d'antigène dont un ligand d'héparane sulfate, un sucre sulfaté de la famille des glycosaminoglycanes. La présente invention concerne également des compositions contenant ledit complexe et leur utilisation comme vaccin.

Lorsque l'on développe des vaccins on cherche à induire de fortes réponses immunitaires car l'efficacité vaccinante est, la plupart du temps, corrélée au niveau de réponse immunitaire préalablement généré. L'aptitude à induire des réponses immunitaires élevées présente trois intérêts supplémentaires. Premièrement, elle peut permettre de limiter le nombre de vaccinations à réaliser pour atteindre le niveau d'immunité requis. Deuxièmement, elle peut permettre de se passer d'adjuvant et ainsi éviter les risques de toxicité liés à leur utilisation. Troisièmement, elle peut permettre de diminuer les doses de vaccin injecté et conduire ainsi à l'obtention d'un plus grand nombre de doses de vaccin pour un même niveau de production. Les avantages multiples procurés par la capacité à induire de fortes réponses immunitaires ont donc conduit de nombreux groupes de recherche à tenter de développer des approches ayant pour but d'accroître l'immunogénicité des antigènes à injecter chez l'animal ou chez l'homme.

La réponse immune est initiée au niveau de cellules présentatrices spécialisées, les cellules présentatrices d'antigène (CPA, CPAg ou APC, pour *Antigen Presenting Cell*)) incluant les cellules dendritiques (CD ou DC pour *Dendritic Cell*). Les CDs expriment une grande variété de molécules de surface dont les molécules du complexe majeur d'histocompatibilité (CMH) qui lient des fragments d'antigène et les présentent aux lymphocytes T. Ces complexes peptide/molécule du CMH sont reconnus par les récepteurs exprimés à la surface des lymphocytes T qui deviennent ainsi activés et contribuent à la réponse immunitaire. On distingue deux types de molécules du CMH : les molécules du CMH de classe I qui présentent l'antigène aux cellules T CD8+ et permettent l'induction de la réponse T cytotoxique, et les molécules du CMH de classe II qui présentent l'antigène à des cellules T CD4+ et contribuent ainsi au déclenchement et au maintien de la réponse humorale. Les antigènes (Ags) apportés de façon exogène aux CPAgs sont apprêtés pour être généralement associés aux molécules du CMH de classe II. Cependant, les CPAgs et, en particulier les CDs, sont aussi capables d'apprêter les antigènes exogènes et de les présenter en association avec les molécules du CMH de classe I selon un processus dit de présentation croisée (Sigal et al., Nature, 1999, 398, 77-80 ; Rock, K.L., Nature Immunology, 2003, 4, 941-943 ; Kasturi, S. P. et Pulendran, B., Nature Immunology, 2008, 9, 461-463). Ainsi, la présentation antigénique restreinte aux molécules du CMH de classe II et la présentation croisée restreinte aux molécules du CMH de classe I jouent un rôle central dans l'initiation et l'amplification des réponses immunitaires humorale et cytotoxique.

De ce fait, un grand nombre d'approches ayant pour objet d'accroître l'immunogénicité des antigènes sont fondées sur l'amélioration de l'efficacité des mécanismes de présentation et de présentation croisée des antigènes. Comme l'efficacité des deux mécanismes de présentation dépend de la capture préalable des Ags exogènes par les CPAgs, ces approches sont principalement fondées sur le ciblage desdits Ags vers les CPAgs. Pour cibler les Ags, on cherche à atteindre sélectivement les CPAgs car l'interaction avec d'autres cellules dépourvues de capacité de présentation pourrait conduire à une dilution de la quantité d'Ag disponible pour les CPAgs et ainsi à une diminution de la réponse immunitaire. Pour atteindre cette sélectivité, on cible des molécules de surface exprimées essentiellement sur les CPAgs. Les récepteurs de surface des CPAgs qui ont été utilisés avec succès comme cible pour augmenter l'immunogénicité des antigènes sont principalement : les molécules du CMH, notamment de classe II; des intégrines (CD11c, MAC1), les récepteurs lectines de type C (récepteur du mannose (CD206), DEC-205(CD205), DC-SIGN (CD209), LOX1, Dectin-1 (récepteur du béta-glucane), Dectin-2, Clec9A, Clec12A, DCIR2, FIRE, CIRE), les immunoglobulines de surface ou immunoglobulines membranaires, les récepteurs pour la région constante des immunoglobulines (FcR ou RFc) et notamment des IgG (RFcy, RFcgamma, FcγR : FcyRI (CD64), FcyRII (CD32), FcyRIII (CD16)), la superfamille des récepteurs du TNF (CD40) et les récepteurs du complément (récepteur du composant C5a ou de C3d (CD21) ; pour une revue voir notamment Keler et al., Oncogene, 2007, 26, 3758-3767 ; Tacken et al., Nature Reviews, Immunology, 2007, 7, 790- ; Shortman et al., Exp. Mol. Med., 2009, 41, 61-66). Le ciblage d'autres récepteurs de CPAgs, notamment le récepteur de protéines de choc thermique (HSR pour *Heat Shock Protein Receptor*) comme la molécule CD91 (Demande Internationale WO 03/084467) et le récepteur de l'interféron-gamma (Demande US 2007/0031445) a également été rapporté. Le ciblage de l'antigène vers les CPAgs est réalisé avec un antigène associé à un ligand de ces molécules de surface de CPAgs.

Ce ligand est généralement un anticorps (Ac) monoclonal spécifique de ces molécules de surface des CPAgs ou un fragment dérivé (F(ab')₂, scFv). Dans ce type de vaccin, dénommé vaccin ciblé par anticorps ou ATV (*Antibody-Targeted Vaccine*), l'Ag est généralement lié à l'Ac en un site distinct du paratope, le plus souvent à l'extrémité C-terminale de la chaîne lourde (région constante) d'un anticorps complet ou de l'une des régions variables (VH ou VL) d'un fragment d'anticorps à chaîne unique (scFv). Le couplage de l'Ag à l'anticorps est, soit un couplage covalent comme un couplage chimique ou une fusion génétique (protéine de fusion), soit un couplage non-covalent par des complexes streptavidine-biotine ou des complexes entre l'Ac et l'Ag couplé ou fusionné à une protéine (protéine A) ou un fragment de protéine qui lie les immunoglobulines. Des vaccins ciblés par anticorps sont notamment décrit dans le Brevet US 6, 365, 161 (Ac anti-FcR), les Demandes Internationales PCT WO 99/29344, WO 00/00156 (Ac anti-CMH II, anti-CD11c), WO 01/64254 (Ac anti-FcR et fragment Fc d'immunoglobulines polyclonales), WO 2008/097866 (Ac anti-DCIR), WO 2008/103953 (Ac anti-LOX1), la Demande US 2004/0258688 (Ac anti-DEC-205), Bozzacco et al., PNAS, 2007, 104, 1289-1294 (Ac anti-DEC-205), Tacken et al., J. Immunol., 2008, 180, 7687-7696 (Ac anti-DC-SIGN), la Demande FR 2 759 296 et Léonetti et al., J. Immunol., 1998, 160, 3820-3827 (Ac anti-CMH II, Ac anti-IgG-F(ab')₂, Ac anti-IgM). Ces vaccins ciblés par anticorps ont montré que le ciblage permet d'augmenter considérablement la capture de l'Ag par les CPAgs et son endocytose dans les compartiments de l'apprêtement ce qui permet d'accroître la présentation antigénique aux cellules T et donc la capacité de l'Ag à stimuler des cellules T et à induire la réponse immunitaire. Le ciblage à l'aide de ligands vers les CPAgs représente donc une voie de choix pour augmenter l'immunogénicité de molécules d'intérêt vaccinant. Ces approches réalisées en présence ou en absence d'adjuvant ont conduit à des résultats prometteurs. Il a notamment été montré que l'on peut induire plus efficacement la réponse CTL restreinte au CMH classe I ainsi que la réponse T auxiliaire restreinte au CMH de classe II. Ces réponses immunitaires permettent d'envisager une amélioration des défenses vis-à-vis des maladies infectieuses et des cancers. Cependant, aucun vaccin n'a, à ce jour, été commercialisé sur la base de ces approches ce qui suggère que la capacité à induire des réponses immunitaires protectrices reste insuffisante.

Alternativement, le ligand est un anticorps polyclonal spécifique de l'antigène, le vaccin étant alors constitué de complexes Ac/Ag (complexes immuns, CI ou IC pour *Immune Complex*), éventuellement associés à une protéine qui lie les immunoglobulines (Ig) comme la protéine A de *S. aureus* (Léonetti et al., J. Exp. Med., 1999, 189, 1217-1228). Différentes études ont montré que des anticorps spécifiques d'un antigène donné peuvent accroître la présentation aux cellules T auxiliaires, en délivrant les complexes immuns aux récepteurs Fc pour les IgG (RFcy ou récepteur Fcgamma) exprimés à la surface des CPAgs (Manca et al., J. Exp. Med., 1991, 173, 37-48; Rouas-Freiss et al., Eur. J. Immunol., 1993, 23, 3335-3344 ; Manca et al., J. Immunol., 1988, 140, 2393-2398 ; Exposito et al., J. Immunol., 1995, 155, 1725-1736). De plus, il a été montré que des complexes immuns liés aux RFcy sur les cellules dendritiques sont présentés par les molécules du CMH classe I à la surface de ces cellules (Regnault et al., J. Exp. Med., 1999, 189, 371-380). Cependant, les approches ciblées sur les RFcy pourraient procurer des effets relativement limités en raison de l'expression des RFcy à la surface de cellules non spécialisées dans la présentation des Ags et parce que le récepteur Fcgamma II présente une isoforme qui inhibe la présentation des complexes immuns (Amigorena et al., Science, 1992, 256, 1808-1812).

D'autres ligands spécifiques de CPAgs ont également été décrits. On peut citer notamment une famille d'exoprotéines de *Staphylococcus aureus* présentant une homologie avec des superantigènes (protéines SSLs pour Staphylococcal *Superantigen-Like exoproteins* ; Demande Internationale PCT WO 2005/092918), les protéines et fragments de protéines qui lient les Ig comme la protéine A de *Staphylococcus aureus* et son dérivé ZZ (Lobeck et al., Inf. Immunity, 1998, 66, 418-423 ; Léonetti et al., J ; Immunol., 1998, 160, 3820-3827), une séquence de ciblage dérivée de la protéine gp96 qui lie CD91 (Demande Internationale WO 03/084467), des Ac monoclonaux recombinants humains liant les cellules dendritiques sélectionnés à partir d'une banque de phages (Demande US 2005/0037001), des particules (fantômes de globules rouges de type O) comprenant un antigène et de l'ATP, recouvertes d'un ligand de CPAgs (IgG, composant C3b ou Cbi du complément, anhydride maléique, lipide oxydé, sucre ou polyanion ; Demande Internationale PCT WO 02/20042), des peptides agoniste de C5a ou analogue de l'interféron-gamma (Demande US 2007/0031445).

La surface des cellules est recouverte de glycolipides et de glycoprotéines (glycocalyx) telles que les protéoglycanes, des glycoprotéines comprenant une ou plusieurs chaînes de glycosaminoglycanes (GAG) non-ramifiées. Les protéoglycanes peuvent être soit transportés à l'extérieur de la cellule par exocytose, s'intégrant alors à la matrice-extracellulaire, sous forme de chondroïtine-sulfate, kératane-sulfate, héparane-sulfate, dermatane-sulfate, soit entrer dans la constitution de la membrane plasmique ou du glycocalyx, jouant alors un rôle dans les relations cellule-matrice. Les héparanes sulfate protéoglycanes sont des molécules ubiquitaires présentes à la surface des cellules de mammifères et dans les matrices extracellulaires (Dreyfuss et al., Annuals of the Brazilian Academy of Sciences, 2009, 81, 409-429). Ces molécules jouent un rôle central dans de nombreux processus biologiques (prolifération cellulaire, adhésion cellulaire, inflammation, coagulation, pénétration cellulaire de microorganismes pathogènes, en particulier des virus et des parasites). Leurs propriétés sont principalement médiées via la partie saccharidique : l'héparane sulfate.

D'une manière générale, le ciblage de molécules qui ne sont pas exprimées sélectivement à la surface des CPAgs n'est pas considéré comme une voie particulièrement efficace car « *in vivo* » elle entraîne une dissémination de l'Ag sur un grand nombre de cellules non spécialisées dans l'induction de la réponse immunitaire ce qui conduit à une diminution de la quantité d'Ag disponible pour les CPAgs. Ce ciblage non-spécifique est considéré comme un inconvénient que l'on cherche à supprimer pour les applications de vaccination *in vivo.*

Par exemple, les vecteurs lentiviraux recombinants pseudotypés avec la glycoprotéine d'enveloppe du virus Sindbis (SVG), qui ciblent la molécule de surface des DCs, DC-SIGN, sont utilisés en vaccination. Toutefois, de tels vecteurs ne permettent pas un ciblage sélectif des DCs du fait que la protéine SVG lie les héparanes sulfates (HS), des molécules de surface ubiquitaires, exprimées sur de nombreux types de cellules de mammifères. Aussi, pour améliorer le ciblage de ces vecteurs-vaccin, la protéine SVG a été mutée pour qu'elle perde sa capacité de liaison des HS (Yang et al., Nat. Biotechnol., 2008, 26, 326-334).

Une approche alternative pour délivrer un antigène dans les CPAgs consiste à lier l'antigène (couplage chimique covalent ou protéine de fusion) à un peptide de pénétration cellulaire (CPP pour *Cell Penetrating Peptides* ; Brooks et al., Biochimica et Biophysica Acta, 2009). Ces peptides naturels ou synthétiques sont, soit des peptides cationiques (riches en lysines et/ou arginines) tels que les peptides dérivés de la région basique de la protéine Tat du VIH (Tat 49-57 : RKKRRQRRR (SEQ ID NO: 1)) et les peptides polyarginines (R7 à R11), soit des peptides basiques/amphiphiles tels que les peptides dérivés de l'homéodomaine de la protéine Antennapedia (pénétratine ; fragment 43-58: RQIKIWFQNRRMKWKK (SEQ ID NO : 2)). Ces peptides sont capables d'être transloqués au travers de la membrane plasmique des cellules, par des mécanismes d'action impliquant ou non l'endocytose médiée par un récepteur glycosaminoglycane (GAG), notamment les héparanes sulfates et les chondroïtines sulfates (Futaki et al., Biopolymers, 6 décembre 2005). Les CPP initialement utilisés pour améliorer la présentation d'Ags exogènes (vaccin) par les CMH I (présentation croisée) et induire une réponse T cytotoxique (Demande Internationale PCT WO 00/35949), sont également capables d'améliorer la présentation des Ags exogènes par les CMH II et de stimuler des lymphocytes T CD4+ (Schutze-Redelmeier et al., J. Immunol., 1996, 157, 650-655 ; Mitsui et al., Journal of Investigative Dermatology, 2006, 126, 1805-1812 ; Wang et al., Immunology Letters, 2009). Des CPP sont également insérés dans un polypeptide de surface d'un vecteur phage pour augmenter l'expression des gènes délivrés par le vecteur dans les cellules de mammifères en favorisant son entrée par les voies indépendantes de la phagocytose et de l'endocytose (WO 2007/015704).

Toutefois, du fait de l'expression ubiquitaire des GAGs, il n'est pas possible de cibler spécifiquement des CPAgs. C'est pourquoi, l'utilisation de CPP comme vaccin est considérée comme une approche valable, uniquement pour la vaccination « *ex vivo* » (Tacken et al., J. Immunol., 2008, 180, 7687-7696). La première étape réalisée « *ex vivo* » consiste en l'isolement de cellules dendritiques du patient puis le chargement en peptides de ces cellules. La seconde étape réalisée « *in vivo* » consiste à injecter les CPAgs chargées avec l'Ag afin de vacciner. Cette approche en deux étapes, qui s'avère relativement efficace pour déclencher des réponses immunitaires protectrices, reste cependant lourde à mettre en place et inadaptée à la vaccination de masse.

Un peptide de Tat comprenant les régions riche en cystéines (Tat 22-37) et core/hydrophobe (Tat 38-48) a un effet adjuvant de la réponse humorale lorsqu'il est couplé de façon covalente à un peptide non-immunogène (Gadzinski et al. (P02-1), Retrovirology, vol. 6, no. Suppl 3, page P6).

L'ensemble de ces observations indique que l'amélioration de la vaccination contre les maladies infectieuses ou les cancers nécessite d'augmenter la réponse immune induite par les candidats vaccins ou par les vaccins existants. Le ciblage des antigènes vers les CPAgs, qui représente une voie de choix pour atteindre cet objectif, devrait cependant être encore optimisé pour être rendu encore plus efficace. Le développement d'approches ciblées optimisées pourrait permettre : l'induction d'effets protecteurs plus importants permettant d'améliorer l'efficacité vaccinale, de limiter le nombre de rappels à effectuer, de diminuer les doses injectées et de se passer d'adjuvant.

Bien que les glycosaminoglycanes de la surface cellulaire tels que les héparanes sulfates (HS) ne semblent pas être des cibles particulièrement adéquates pour permettre l'accroissement d'une réponse immunitaire spécifique, les inventeurs ont découvert que leur ciblage peut permettre une augmentation significative, voire même avoir un effet synergique, sur la réponse immunitaire spécifique d'immunogènes qui sont associés à un ligand capable de cibler sélectivement les CPAgs. Pour évaluer cet aspect, les inventeurs ont utilisé deux constructions différentes qui contiennent chacune un Ag, un ligand des HS et un ligand d'un récepteur de CPAg. Pour ces deux types de constructions, les inventeurs ont observé que la présence du double ciblage procure une synergie d'effet et permet ainsi une augmentation de la réponse immunitaire qui est mesurée à l'aide d'un test « *in vitro »* de stimulation de cellules T spécifiques de chacun des deux Ags étudiés. L'analyse par cytométrie de flux a permis aux inventeurs d'observer que les deux constructions se lient de façon privilégiée et accrue aux CPAgs. Ces observations indiquent donc que l'association d'un ligand de récepteur spécifique et d'un ligand de molécule ubiquitaire, tels que les HS, permet d'accroître la réponse immunitaire spécifique d'Ags. Il n'était pas évident que la combinaison du ciblage des HS et de protéines de surface spécifiques des CPAgs puisse augmenter et encore moins qu'elle puisse produire une synergie d'effet sur la réponse immunitaire spécifique de l'antigène (la présentation de l'antigène est augmentée d'un facteur 700 dans le cas de la toxine alpha). Cette synergie d'effet était d'autant moins évidente en raison de l'expression ubiquitaire des HS. En effet, les composés double-ciblés auraient pu être répartis sur des CPAgs ainsi que sur d'autres cellules incapables de présenter l'Ag ce qui aurait pu conduire à une diminution de la réponse par rapport à des composés ciblés uniquement sur des protéines de CPAg. Ce système de ciblage multiple permet d'augmenter l'immunogénicité obtenue par simple ciblage.

En conséquence, la présente invention a pour objet un complexe moléculaire tel que défini dans les revendications.

### Définitions

- On entend par « antigène » toute substance pouvant être reconnue spécifiquement par le système immunitaire et en particulier par les anticorps et les cellules du système immunitaire (lymphocytes B, lymphocytes T CD4+, lymphocytes T CD8+).
- On entend par « réponse immunitaire spécifique d'un antigène », la production d'anticorps, et/ou l'induction d'une réponse T auxiliaire (activation de lymphocytes T CD4+), et/ou T cytotoxique (activation de lymphocytes T CD8+) spécifiques dudit antigène. Cette réponse peut notamment être mesurée, *in vitro,* par un test de stimulation de lymphocytes T CD4+ ou T CD8+ spécifiques de l'antigène. Dans ce test, les lymphocytes T spécifiques sont incubés en présence de cellules présentatrices d'antigène autologues vivantes et de l'antigène.
- On entend par « immunogénicité » d'un antigène sa capacité à induire une réponse immunitaire spécifique.
- On entend par « cellule présentatrice d'antigène(s) », cellule présentatrice de l'antigène, CPA, CPAg ou APC pour *Antigen Presenting Cell,* une cellule exprimant une ou plusieurs molécules du complexe majeur d'histocompatibilité (CMH) de classe I et de classe II (molécules HLA de classe I et de classe II chez l'homme) et capable de présenter les antigènes aux lymphocytes T CD4+ et T CD8+ spécifiques de cet antigène. Comme cellules présentatrices d'antigène, on peut citer notamment les cellules dendritiques (CD ou DC pour *Dendritic Cell*), les cellules mononuclées du sang périphérique (PBMC), les monocytes, les macrophages, les lymphocytes B, les lignées lymphoblastoïdes, et les lignées de cellules humaines ou animales génétiquement modifiées exprimant des molécules du CMH de classe I et de classe II, notamment des molécules HLA I et HLA II.
- On entend par « molécule de surface de cellules présentatrices d'antigène », une molécule exprimée à la surface de cellules présentatrices d'antigène.
- On entend par « molécule de surface spécifique des cellules présentatrices d'antigène », une molécule possédant une spécificité d'expression élevée pour les CPAgs, à savoir une molécule de surface exprimée sur un nombre très limité de cellules autres que les CPAgs, c'est-à dire une molécule de surface exprimée essentiellement sur les cellules présentatrices d'antigène.
- On entend par « apprêtement de l'antigène » ou « *processing* de l'antigène », les transformations que subit l'Ag et qui lui permettent de passer d'une forme incapable de se lier aux molécules du CMH à une forme capable de se lier. Ces étapes de préparation de l'antigène par les CPAgs vivantes, aboutissent donc à la formation de complexes peptide/molécule du CMH I ou peptide/molécule du CMH II. Les complexes sont ensuite exprimés à la surface des CPAgs. Pour être apprêté, généralement, l'Ag doit tout d'abord être endocyté ou phagocyté par des CPAgs. L'apprêtement se déroule ensuite principalement dans les vésicules d'endocytose et dans le cytoplasme des CPAgs. Dans ces compartiments, l'Ag subit une dégradation protéolytique et/ou une dénaturation liée au pH ou à des oxydoréductases, s'en suivent le chargement des peptides sur les molécules du CMH de classe I et de classe II et le transport des complexes peptide/CMH à la surface des CPAgs.
- On entend par « récepteur d'endocytose », un récepteur capable de médier l'endocytose d'un ligand de ce récepteur.
- On entend par glycosaminoglycane (GAG) un polysaccharide linéaire composé de la répétition d'un diholoside contenant toujours une hexosamine (glucosamine (GlcN) ou galactosamine (GaIN)) et un autre ose (acide glucuronique (GlcA), acide iduronique (IdoA), galactose (Gal)). La glucosamine est soit N-sulfatée (GlcNS), soit N-acétylée (GlcNac). La galactosamine est toujours N-acétylée (GalNac). Les glycosaminoglycanes sulfatés sont notamment des polymères simples de GlcA tel que le chondroïtine sulfate et des copolymères comprenant à la fois des résidus de GlcA et/ou d'IdoA et/ou de Gal, tels que l'héparine, l'héparane sulfate, le dermatane sulfate, et le kératane sulfate. Les chaînes de GAG peuvent être liées de façon covalente à des protéines (protéoglycanes) qui sont exprimées à la surface des cellules de mammifères et/ou sécrétées dans le milieu extracellulaire.
- On entend par anticorps, une immunoglobuline (IgG, IgM, IgA, IgD, IgE). Le terme anticorps inclut un anticorps spécifique, c'est à dire un anticorps dirigé contre une molécule x particulière (anticorps anti-molécule x), et un anticorps non-spécifique. Un anticorps spécifique est notamment un anticorps dirigé contre un antigène (anticorps anti-antigène) ou une molécule de surface de CPAg (anticorps anti-molécule de surface de CPAg).
- On entend par épitope, la séquence d'un antigène qui est reconnue par un anticorps (épitope B), un lymphocyte T CD4+ (épitope T CD4+) ou un lymphocyte T CD8+ (épitope T CD8+), spécifique de cet antigène. Il s'agit notamment d'un peptide (peptide épitope).

Le complexe moléculaire selon l'invention comprend au moins : un antigène associé à au moins deux ligands de molécules de surface de CPAgs. Dans ce complexe, le premier ligand qui cible un héparane sulfate est lié de façon covalente à l'antigène, et éventuellement au deuxième ligand.

La liaison covalente est notamment générée par un couplage chimique covalent (formation d'un conjugué covalent) ou par la construction d'une protéine de fusion (fusion génétique).

Conformément à l'invention, les ligands sont des ligands hétérologues, c'est-à-dire que l'antigène et les ligands sont issus de trois molécules initiales différentes ou lorsque l'antigène et l'un des ligands ou les deux ligands sont issus de la même molécule initiale, la protéine de fusion selon l'invention a une séquence qui est différente de celle la molécule initiale. La protéine de fusion selon l'invention comprend au moins une mutation (insertion, délétion, substitution) par rapport à la séquence de la molécule initiale.

Le complexe moléculaire selon l'invention peut être un complexe entièrement covalent dans lequel le premier ligand, le second ligand et l'antigène sont liés, soit uniquement par des couplages chimiques covalents ou des fusions génétiques, soit par un mélange des deux, l'une des liaisons étant générée par fusion génétique et l'autre par un couplage chimique covalent. Par exemple, le premier ligand est lié de façon covalente à l'antigène et le deuxième ligand est lié de façon covalente, soit à L1, soit à l'Ag. Il s'agit notamment d'un conjugué covalent L1-Ag-L2-ou L2-L1-Ag ou d'une protéine de fusion L1-Ag-L2, L2-Ag-L1, L2-L1-Ag ou Ag-L1-L2.

Le complexe moléculaire selon l'invention peut être aussi un complexe mixte dans lequel la liaison du deuxième ligand à L1-Ag (conjugué covalent L1-Ag ou protéine de fusion L1-Ag ou Ag-L1) est non-covalente. La liaison non-covalente est obtenue par tout moyen connu de l'Homme du métier. Elle peut notamment être obtenue en utilisant une molécule (élément de liaison), notamment un peptide, possédant une affinité élevée et spécifique pour L1, L2 ou l'Ag. Cet élément de liaison est lié de façon covalente à l'antigène (Ag), au deuxième ligand (L2), ou à L1-Ag, pour associer de façon non-covalente, le deuxième ligand à L1-Ag. L'affinité de l'élément de liaison pour son partenaire, dans le complexe L1-Ag/L2-élément de liaison, est suffisante pour qu'il ne se dissocie pas immédiatement de ce complexe *in vivo.* Lorsque l'un des ligands est un anticorps, l'élément de liaison est notamment une protéine ou un fragment de protéine qui lie la région Fc et/ou Fab des immunoglobulines, tels que décrits dans la Demande FR 2759296. De tels éléments de liaison aux immunoglobulines incluent notamment la protéine A de *S. aureus,* son fragment BB (SEQ ID NO : 4) et son dérivé ZZ (SEQ ID NO : 3), les deux premières protéines liant les régions Fc et le Fab des immunoglobulines alors que ZZ ne lie que la région Fc. Par exemple, lorsque L2 est un anticorps, l'élément de liaison aux immunoglobulines est lié de façon covalente (couplage chimique covalent ou protéine de fusion), soit à l'antigène, soit à L1 lié de façon covalente à l'antigène (L1-Ag).

L'antigène est éventuellement associé, de façon covalente ou non covalente, à d'autres ligands de molécules de surface de CPAgs, notamment à des ligands qui ciblent les mêmes molécules que le premier ou le deuxième ligand ou bien une ou plusieurs molécules différentes des molécules précédentes.

Le complexe moléculaire selon l'invention se lie au moins à un héparane sulfate et à une molécule de surface exprimée essentiellement sur les CPAgs, puis le complexe est internalisé par les CPAgs et l'antigène est apprêté par les CPAgs pour être présenté par les molécules du CMH (classe I et/ou classe II) à la surface des CPAgs. L'antigène associé séparément à chacun des ligands se lie à son récepteur de surface exprimé sur les CPAgs (simple ciblage des CPAgs). De préférence, l'un au moins des complexes antigène/premier ligand et antigène/second ligand, formé séparément, de manière préférée le complexe antigène/second ligand, est internalisé par les CPAgs et l'antigène est apprêté par les CPAgs pour être présenté par les molécules du CMH (classe I et/ou classe II) à la surface des CPAgs. De manière encore plus préférée, chacun des complexes ainsi formé séparément (antigène/premier ligand et antigène/second ligand), est internalisé par les CPAgs et l'antigène est apprêté par les CPAgs pour être présenté par les molécules du CMH (classe I et/ou classe II) à la surface des CPAgs.

L'apprêtement de l'antigène (libre ou complexé à l'un des deux ligands ou aux deux) par les CPAgs et sa présentation par les molécules du CMH (classe I et/ou classe II) à la surface des CPAgs peuvent être mesurés *in vitro,* par un test de stimulation de lymphocytes T CD4+ ou T CD8+ spécifiques de l'antigène en présence de cellules présentatrices d'antigène autologues vivantes préalablement mises en contact avec ledit antigène (libre ou complexé à l'un des deux ligands ou aux deux). La capacité T stimulante de l'antigène libre ou complexé à l'un des deux ligands ou aux deux peut ainsi être mesurée et comparée. La capacité T stimulante de l'antigène est significativement augmentée avec le complexe selon l'invention, par rapport à l'antigène libre ou l'antigène associé à un seul des ligands.

L'antigène est une protéine ou un peptide, il s'agit de préférence d'un antigène vaccinal tel qu'un antigène spécifique d'un agent pathogène (virus (VIH, Influenza,..), bactérie, parasite, champignon,..) ou d'une tumeur. Il s'agit d'un antigène naturel, recombinant ou synthétique, notamment d'un agent pathogène atténué ou inactivé (virus, bactérie) de particules virales synthétiques, d'une molécule isolée (protéine,) ou de fragments de molécules comprenant un ou plusieurs épitopes B, T CD4+, T CD8+, notamment sous forme de peptides ou de polypeptides comprenant des épitopes issus d'un seul antigène ou de plusieurs antigènes différents (fragment polyépitopique).

Le sucre sulfaté de la famille des glycosaminoglycanes exprimé à la surface des CPAgs (GAG sulfaté) qui est ciblé par le premier ligand est un héparane sulfate.

Le premier ligand est issu d'une cellule de mammifère ou d'un microorganisme pathogène, notamment un virus (Adénovirus, Cytomégalovirus, VIH, Virus Sindbis), une bactérie *(Mycobacterium bovis, Bordetella pertussis),* un parasite (*Leishmania sp*.) ou une toxine, il s'agit d'une protéine ou un de ses fragments qui lie les héparanes sulfates et qui de préférence utilise les protéoglycanes à GAG sulfaté comme récepteur d'endocytose.

De tels ligands sont notamment décrits dans « Heparan Binding Proteins ", H. Edward Conrad, Academic Press, San Diego and Lond*on* et Dreyfuss et al., Annuals of the Brazilian Academy of Sciences, 2009, 81, 409-429 (voir notamment le Tableau II). Des exemples de ces ligands incluent de façon non-limitative : la protéine Tat du VIH et ses fragments, en particulier les fragments comprenant uniquement la région basique de Tat (Tat 49-57 : RKKRRQRRR : SEQ ID NO : 1) ou la région basique et la région centrale de Tat (cette région centrale ou core consiste en Tat 38-48 : FTKKGLGISYG : SEQ ID NO : 5) ; les dodécaèdres dérivés du penton de l'Adénovirus (Vivès et al., Virology, 2004, 321 : 332-340) ; la protéine d'enveloppe du VIH ou la région V3 de cette protéine (Roderiquez et al., J. Virol., 1995, 69, 2233-), la glycoprotéine d'enveloppe du virus Sindbis (Byrnes, A.P. et Griffin, D.E., J. Virol., 1998, 2, 7349-7356) et le domaine R de la toxine diphtérique (fragment 382 à 535 (SEQ ID NO : 6) ; Lobeck et al., Infection and Immunity, 1998, 66, 418-423). On peut citer également les peptides de pénétration cellulaire (*CPP pour Cell Penetrating Peptide*) qui lient les héparanes sulfates tels que les peptides très riches en résidus basiques, en particulier en arginines qui incluent les peptides dérivés de la région basique de la protéine Tat du VIH (Tat 49-57), précités, et les peptides polyarginines (R7 à R11), ainsi que les peptides basiques/amphiphiles tels que les peptides dérivés de l'homéodomaine de la protéine Antennapedia (pénétratine ; fragment 43-58: RQIKIWFQNRRMKWKK (SEQ ID NO : 2)).

Alternativement, le premier ligand est un anticorps naturel ou recombinant dirigé contre les héparanes sulfates ou un fragment de cet anticorps contenant au moins le paratope (domaine de liaison à l'antigène) tel qu'un fragment Fab, Fab', F(ab')₂, Fv ou Fv simple chaîne (scFv pour *single-chain Fv*), Fabc, et fragment Fab comprenant une portion du domaine Fc. De tel anticorps sont notamment décrits dans Thompson et al., J. Biol. Chem., 2009, 284, 35621-35631 et van Kuppevelt et al., J. Biol. Chem., 1998, 273, 12960-12966. De préférence, ledit anticorps ou fragment d'anticorps est humain ou humanisé.

Selon un mode de réalisation avantageux du complexe moléculaire selon l'invention, le ligand d'héparane sulfate exprimé à la surface des CPAgs est un peptide sélectionné dans le groupe constitué par : un fragment de la protéine Tat du VIH, comprenant au moins la région basique (Tat 49-57) tel que les peptides Tat 49-57 (SEQ ID NO: 1) et Tat 37-57 (CFTKKGLGISYGRKKRRQRRR : SEQ ID NO : 7), un peptide polyarginine R7 à R11, et un peptide comprenant le domaine R de la toxine diphtérique (fragment 382 à 535 : SEQ ID NO : 6).

La molécule exprimée à la surface des CPAgs qui est ciblée par le deuxième ligand et éventuellement les autres ligands (troisième,..) est une molécule de surface exprimée essentiellement sur les CPAgs et en particulier sur les cellules dendritiques. De préférence, ladite molécule de surface des CPAgs est un récepteur d'endocytose. Parmi ces molécules de surface, on peut citer notamment : les molécules du CMH (classe I et II), les immunoglobulines de surface, des intégrines (CD11c, MAC1), les récepteurs de la transferrine, les récepteurs des lectines de type C (récepteur du mannose (CD206), DEC-205(CD205), DC-SIGN (CD209), LOX1, Dectin-1 (récepteur du béta-glucane), Dectin-2, Clec9A, Clec12A, DCIR2, FIRE, CIRE), les récepteurs pour la région constante des immunoglobulines (FcR et notamment FcγR : FcγRI (CD64), FcyRII (CD32), FcyRIII (CD16)), la superfamille des récepteurs du TNF (CD40) et les récepteurs du complément.

Selon un autre mode de réalisation avantageux du complexe moléculaire selon l'invention, la molécule exprimée à la surface des CPAgs qui est ciblée par le deuxième ligand et éventuellement les autres ligands est sélectionnée dans le groupe constitué par : les molécules du CMH de classe II, les récepteurs des lectines de type C, les immunoglobulines et les récepteurs pour la région constante des immunoglobulines (RFc ou FcR).

Le deuxième ligand et éventuellement les autres ligands sont notamment des ligands naturels de ces molécules de surface des CPAgs choisis parmi des immunoglobulines et leurs fragments comprenant la région constante qui lient les FcR, et des protéines ou des fragments de protéines qui lient la région Fc et/ou ou Fab des immunoglobulines, tels que décrits dans la Demande FR 2759296 (immunoglobuline de surface) qui utilisent ces molécules de surface des CPAgs comme récepteur d'endocytose. Alternativement, le deuxième ligand et éventuellement les autres ligands sont des anticorps naturels ou recombinants dirigés contre ces molécules de surface des CPAgs ou des fragments de ces anticorps contenant au moins le paratope (domaine de liaison à l'antigène), tels que les fragments Fab, Fab', F(ab')₂, Fv ou Fv simple chaîne (scFv pour *single-chain Fv*), Fabc, et fragment Fab comprenant une portion du domaine Fc. De préférence, ledit anticorps ou fragment d'anticorps est humain ou humanisé.

Selon une disposition avantageuse des modes de réalisation précédents, le complexe selon l'invention comprend : (i) un antigène lié de façon covalente à un peptide ligand de GAG sulfaté tel que défini ci-dessus (premier ligand) (ii) un anticorps dirigé contre ledit antigène, de préférence une IgG (deuxième ligand), et éventuellement une protéine ou un fragment de protéine qui lie la région Fab des anticorps telle que la protéine A de *Staphylococcus aureus.*

Selon une autre disposition avantageuse des modes de réalisation précédents, le complexe selon l'invention comprend : (i) un antigène lié de façon covalente à un peptide ligand d'héparane sulfate tel que défini ci-dessus (premier ligand), et à une protéine ou un fragment de protéine qui lie la région Fc et/ou Fab des anticorps, de préférence uniquement la région Fab, tel que la protéine A de *Staphylococcus aureus* et son fragment BB (élément de liaison), (ii) un anticorps (non-spécifique ou spécifique dudit antigène), de préférence une IgG, ou un fragment dudit anticorps comprenant au moins la région Fc (deuxième ligand), et éventuellement une protéine ou un fragment de protéine qui lie la région Fab des anticorps telle que la protéine A de *S.aureus,* lorsque l'antigène n'est pas déjà lié à celle-ci. De préférence, l'anticorps est une immunoglobuline complète et l'antigène est lié de façon covalente à une protéine ou un fragment de protéine qui lie uniquement la région Fab des anticorps, tel que la protéine A de *S. aureus* et son fragment BB.

Selon une autre disposition avantageuse des modes de réalisation précédents, le complexe selon l'invention comprend un antigène lié de façon covalente à un peptide ligand d'héparane sulfate tel que défini ci-dessus (premier ligand) et à une protéine ou un fragment de protéine qui lie la région Fc et/ou Fab des immunoglobulines tel que le fragment BB de la protéine A de *Staphylococcus aureus* et son dérivé ZZ (deuxième ligand).

Selon une autre disposition avantageuse des modes de réalisation précédents, le complexe selon l'invention comprend un antigène lié de façon covalente à un peptide ligand d'héparane sulfate tel que défini ci-dessus (premier ligand) et à un anticorps sélectionné dans le groupe constitué par un anticorps anti-CMH de classe II, un anticorps anti-RFcgamma (I, II, et/ou III) et un anticorps anti-DEC-205, ou bien à un fragment des anticorps précédents comprenant au moins le paratope tel que par exemple, un fragment Fab, Fab', F(ab')₂, Fv, scFv, Fabc ou Fab comprenant une portion de la région Fc (deuxième ligand).

Selon encore une autre disposition avantageuse des modes de réalisation précédents, le complexe selon l'invention comprend : (i) un antigène lié de façon covalente à un peptide ligand d'héparane sulfate tel que défini ci-dessus (premier ligand) et à une protéine ou un fragment de protéine qui lie la région Fc et/ou Fab des immunoglobulines tel que le fragment BB de la protéine A de *Staphylococcus aureus* et son dérivé ZZ (élément de liaison), et (ii) un anticorps sélectionné dans le groupe constitué par un anticorps anti-CMH de classe II, un anticorps anti-RFcgamma (I, II et/ou III), et un anticorps anti-DEC-205, ou un fragment des anticorps précédents comprenant au moins le paratope, notamment un fragment Fab, Fab', F(ab')₂, Fv, scFv, Fabc ou Fab comprenant une portion de la région Fc (deuxième ligand).

L'Homme du métier a bien connaissance des spécificités d'espèce des biomolécules et notamment des protéines. Par conséquent, l'homme du métier reconnaîtra aisément l'avantage de cibler les molécules de surface de la même espèce que l'espèce à immuniser et d'utiliser des ligands de la même espèce que l'espèce à immuniser ou dont la séquence a été adaptée à cette espèce. Par exemple, pour préparer un vaccin humain, il est préférable d'utiliser des anticorps humains ou humanisés dirigés contre une molécule de surface humaine.

La présente invention a également pour objet un vecteur recombinant isolé ou un mélange de vecteurs recombinants comprenant les séquences codant pour l'antigène, le premier et le second ligand, insérées dans un polynucléotide, une construction polynucléotidique, isolés ou bien un mélange de polynucléotides ou de constructions polynucléotidiques, sélectionnés dans le groupe constitué par :
a) un polynucléotide isolé comprenant une séquence codant pour une protéine de fusion comprenant au moins les séquences codantes de l'antigène, du premier et du second ligand, fusionnées en phase de façon appropriée,
b) un mélange de polynucléotides comprenant au moins un premier polynucléotide comprenant une séquence codant pour une protéine de fusion comprenant au moins les séquences codantes de l'antigène et du premier ligand fusionnées en phase de façon appropriée et un second polynucléotide comprenant une séquence codant pour le deuxième ligand, ledit premier ou second nucléotide comprenant également une séquence codant pour un élément de liaison tel que défini ci-dessus,
c) une construction polynucléotide isolée ou un mélange de constructions polynucléotidiques comprenant au moins le polynucléotide tel que défini en a) ou les deux polynucléotides tels que définis en b), liés de façon opérationnelle à des séquences régulatrices de la transcription et/ou de la traduction appropriées (promoteur, activateur de transcription, terminateur de transcription, signal de polyadénylation) pour l'expression du premier ligand, de l'antigène et/ou du second ligand dans des cellules hôtes.

Les polynucléotides selon l'invention codent pour des antigènes ou des ligands qui sont des protéines ou des peptides, éventuellement des glyco-ou lipo-peptides ou protéines.

Le mélange de vecteurs recombinants comprend au moins un premier vecteur comprenant le premier polynucléotide ou la construction polynucléotide dérivée tels que définis en b) et c) et un deuxième vecteur comprenant le deuxième polynucléotide ou la construction polynucléotide dérivée tels que définis en b) et c).

De nombreux vecteurs dans lesquels on peut insérer un polynucléotide d'intérêt afin de l'introduire et de le maintenir dans une cellule hôte eucaryote sont connus en eux-mêmes ; le choix d'un vecteur approprié dépend de l'utilisation envisagée pour ce vecteur (par exemple réplication de la séquence d'intérêt, expression de cette séquence, maintien de la séquence sous forme extrachromosomique ou bien intégration dans le matériel chromosomique de l'hôte), ainsi que de la nature de la cellule hôte. On peut utiliser entre autres, des vecteurs viraux (adénovirus, rétrovirus, lentivirus, AAV) et des vecteurs non-viraux (ADN-nu), notamment un plasmide, dans lesquels a été insérée préalablement la séquence d'intérêt.

De préférence, le ou lesdits vecteurs recombinants sont des vecteurs d'expression comprenant au moins une construction polynucléotidique telle que définie ci-dessus. Les vecteurs d'expression sont utiles pour la production du complexe selon l'invention dans des cellules hôtes appropriées.

De manière préférée, le vecteur recombinant selon l'invention est un plasmide d'expression, qui est utile pour la production du complexe selon l'invention.

La demande décrit des cellules procaryotes ou eucaryotes transformées par un vecteur recombinant tel que défini ci-dessus.

La présente invention a également pour objet un complexe moléculaire selon l'invention, comme vaccin pour la prévention ou le traitement d'une maladie infectieuse ou d'un cancer.

La composition immunogène ou vaccinale comprend au moins un complexe moléculaire tel que défini ci-dessus et un véhicule pharmaceutiquement acceptable.

La composition vaccinale comprend une quantité de complexe(s), polynucléotide(s) suffisante pour induire une réponse immunitaire spécifique d'un pathogène ou d'une tumeur capable de protéger contre l'infection par ce pathogène ou d'en atténuer les conséquences ou bien de réduire la croissance d'une tumeur, chez un individu vacciné avec cette composition.

Les véhicules pharmaceutiquement acceptables sont ceux classiquement utilisés.

La composition vaccinale comprend, éventuellement, également un adjuvant de l'immunité humorale et/ou cellulaire. Les adjuvants sont avantageusement choisis dans le groupe constitué par : des émulsions huileuses, des substances minérales, des extraits bactériens, la saponine, l'hydroxyde d'alumine, le mono-phosphoryl -lipide A, le squalène et les ligands de TLR, notamment les oligonucléotides comprenant au moins une séquence CpG qui sont des ligands de TLR9.

La composition vaccinale selon l'invention se présente sous une forme galénique adaptée à une administration par voie parentérale (sous-cutanée, intramusculaire, intraveineuse), entérale (orale, sublinguale) ou locale (rectale, vaginale).

De préférence, ladite composition comprend également une substance porteuse. Les substances porteuses sont celles classiquement utilisées. Il s'agit notamment de liposomes unilamellaires ou multilamellaires, d'ISCOMS, de virosomes (*virus-like particles*), de micelles de saponine, de microsphères solides de nature saccharidique (poly(lactide-co-glycolide)) ou aurifère, et de nanoparticules.

La composition vaccinale peut comprendre plusieurs complexes moléculaires comprenant des antigènes et/ou des ligands différents ou bien au moins un autre antigène d'intérêt (non complexé à des ligands selon l'invention), tels que définis ci-dessus. Le ou lesdits complexes sont éventuellement liés entre eux par des liaisons covalentes ou non-covalentes et/ou incorporés à l'intérieur ou à la surface d'une particule telle qu'un liposome ou un virosome.

Les complexes de ciblage de l'antigène selon l'invention sont préparés par les techniques classiques connues de l'Homme du métier, à savoir :
- l'antigène, et les ligands des molécules de surface des CPAgs peuvent être produits par synthèse chimique ou par expression d'un ADN recombinant dans un système cellulaire approprié, eucaryote ou procaryote. Les peptides et protéines peuvent être synthétisés en phase solide, selon la technique Fmoc, originellement décrite par Merrifield et al. (J. Am. Chem. Soc., 1964, 85, 2149-) et purifiés par chromatographie liquide haute performance en phase inverse. Les polypeptides et les protéines peuvent être produits à partir des ADNc correspondants, clonés dans un vecteur d'expression eucaryote ou procaryote approprié, les polypeptides ou protéines produits dans les cellules modifiées par le vecteur recombinant sont purifiés par tout moyen approprié, notamment par chromatographie d'affinité. Des anticorps dirigés contre des molécules de surface de CPAgs sont bien connus et disponibles commercialement. A titre d'exemple non-limitatif, les anticorps anti-CD205 (#555831) ; anti-CD206 (#555952) ; anti-CD209 (#551186) ; anti-HLA-DR (#555556) sont disponibles chez BECTON-DICKINSON . Alternativement, des anticorps monoclonaux peuvent être produits par les techniques classiques connues de l'homme du métier. Par exemple, les anticorps monoclonaux sont produits à partir d'hybridomes obtenus par fusion de lymphocytes B d'un animal immunisé par la molécule de surface de CPAgs avec des myélomes, selon la technique de Köhler et Milstein (Nature, 1975, 256, 495-497) ; les hybridomes sont cultivés *in vitro,* notamment dans des fermenteurs ou produits in vivo, sous forme d'ascite ; alternativement lesdits anticorps monoclonaux sont produits par génie génétique comme décrit dans le brevet américain US 4,816,567. Les anticorps humanisés sont produits par des méthodes générales comme celles décrites dans la Demande Internationale WO 98/45332. Les fragments d'anticorps sont produits à partir des régions V_{H} et V_{L} clonées, à partir des ARNm d'hybridomes ou de lymphocytes spléniques d'un mammifère immunisé; par exemple, les fragments Fv, scFv ou Fab sont exprimés à la surface de phages filamenteux selon la technique de Winter et Milstein (Nature, 1991, 349, 293-299); après plusieurs étapes de sélection, les fragments d'anticorps spécifiques de l'antigène sont isolés et exprimés dans un système d'expression approprié, par les techniques classiques de clonage et d'expression d'ADN recombinant. Les anticorps ou leur fragments tels que définis ci-dessus, sont purifiés par les techniques classiques connues de l'Homme du métier, telles que la chromatographie d'affinité.
- l'association covalente du premier ligand (L1) à l'antigène (Ag), et éventuellement au deuxième ligand (L2) d'une molécule de surface des CPAgs, peut-être réalisé par la construction d'une protéine de fusion dans laquelle les séquences nucléotidiques codant pour L1, l'Ag, et éventuellement L2 sont fusionnées en phase, dans l'ordre approprié, soit directement, soit par l'intermédiaire d'une séquence nucléotidique codant pour un peptide de liaison (*peptide linker*) approprié. En fonction des tailles respectives des séquences en acides aminés de L1, l'Ag, et éventuellement L2, elles sont soit fusionnées au niveau de leurs extrémités (extrémité N-terminale de l'une des séquences fusionnée à l'extrémité C-terminale de l'autre séquence) ou l'une des séquences est insérée dans l'autre séquence à un site approprié qui n'a pas d'effet délétère sur l'immunogénicité de l'antigène et la liaison du ou des ligands à son récepteur exprimé à la surface des CPAgs. Alternativement, l'antigène et le(s) ligand(s) peuvent être couplés de façon covalente, par tout moyen approprié. Le couplage est effectué par l'intermédiaire de groupements réactionnels initialement présents ou préalablement introduits sur l'antigène et le(s) ligand(s). Le couplage peut notamment être réalisé au niveau de résidus d'acides aminés dont la chaîne latérale comprend une fonction réactive. Parmi ces acides aminés, on peut citer les acides aminés polaires comprenant une fonction : -OH [sérine (S), thréonine (T) ou tyrosine (Y)], -SH [cystéine (C)], -NH₂ [lysine (K) ou arginine (R)], -COOH [acide aspartique (D) ou acide glutamique (E)], et les acides aminés polaires à chaîne latérale fonctionnalisée par addition d'une fonction réactive, notamment un chloro- ou bromo-acétyl réactif avec les groupements thiol ou un groupement hydrazine réactif avec les aldéhydes. L'antigène est couplé au ligand par tout moyen approprié ; ces moyens qui sont connus de l'Homme du métier incluent notamment le couplage à l'aide de réactifs homobifonctionnels comme le glutaraldéhyde ou le dithiobis-(succinimidyl propionate). De préférence, le couplage est réalisé à l'aide de réactifs hétérobifonctionnels, notamment le m-maleimidobenzoyl-N-hydroxysuccinimide (SMCC) ou le sulfo-SMCC qui contiennent chacun un groupement maléimide capable de réagir avec les thiols libres. Dans ce cas, le SMCC est préalablement lié de façon covalente à une fonction amine présente sur l'Ag ou le ligand. Dans le même temps, un autre réactif hétérobifonctionnel (comme le N-succinimidyl S-acetylthioacétate qui contient un groupement thio-ester clivable à l'hydroxylamine ou le succinimidyl-pyridyl-dithiopropionate, qui contient un pont disulfure réductible en conditions douce), est associé à une fonction amine du second partenaire qui est soit l'Ag soit un des ligands. Le second partenaire est ensuite traité à l'hydroxylamine ou avec un réducteur pour permettre la libération du thiol. Le composé thiolé est alors incubé avec le composé ayant incorporé le maléimide et le couplage est obtenu par réaction du groupement thiol sur le groupement maléimide. Ce type de couplage covalent est notamment décrit dans Léonetti et al., J. Exp. Med., 1999, 189, 1217-1228. Il est aussi possible de libérer un groupement thiol déjà présent sur un des composés pour ensuite réaliser son couplage à un autre composé qui a été préalablement modifié à l'aide de SMCC. Cette méthode, qui est souvent employée pour coupler des anticorps à des ligands, est notamment décrite dans Ishikawa et al., J. Immunoassay, 1983, 4, 209-327.
- les complexes non-covalents sont préparés par mise en contact du deuxième ligand (L2) avec l'antigène lié de façon covalente au premier ligand (L1-Ag, l'ordre de L1 et de l'Ag étant indifférent lorsqu'il s'agit d'une protéine de fusion), dans des conditions permettant aux deux partenaires d'interagir. Cette interaction peut impliquer un élément de liaison, notamment une protéine ou un peptide, qui possède une affinité élevée et spécifique, pour l'un des partenaires du complexe (Ag, L1 ou L2). En particulier, l'affinité de l'élément de liaison pour ce partenaire, dans le complexe, est suffisante pour qu'il ne se dissocie pas immédiatement de ce complexe *in vivo.* Lorsque l'un des ligands est une immunoglobuline, l'élément de liaison est un élément de liaison aux immunoglobulines tel que décrit dans la Demande FR 2759296. Par exemple, un élément de liaison aux immunoglobulines est lié de façon covalente à l'antigène lié de façon covalente à L1 (L1-Ag), de façon à former un complexe non-covalent avec L2.
- les polynucléotides codants pour l'antigène, le premier et le second ligand, tels que définis ci-dessus sont obtenus par les méthodes classiques, connues en elles-mêmes, en suivant les protocoles standards tels que ceux décrits dans Current Protocols in Molecular Biology (Frederick M. AUSUBEL,2000, Wiley and son Inc, Library of Congress, USA*).* Par exemple, ils peuvent être obtenus par amplification d'une séquence nucléique par PCR ou RT-PCR, par criblage de banques d'ADN génomique par hybridation avec une sonde homologue, ou bien par synthèse chimique totale ou partielle. Les vecteurs recombinants sont construits et introduits dans des cellules hôtes par les méthodes classiques d'ADN recombinant et de génie génétique, qui sont connues en elles-mêmes.

La mise en oeuvre de l'invention utilise, sauf indication contraire, des méthodes classiques d'immunologie, de culture cellulaire, de biologie cellulaire, de biologie moléculaire et d'ADN recombinant qui sont connues de l'Homme du métier. Ces techniques sont décrites en détail dans la littérature, se référer par exemple à: Current Protocols in Molecular Biology (Frederick M. AUSUBEL, 2000, Wiley and Sons Inc, Library of Congress, USA); Current Protocols in Immunology (John E. Coligan et al., 2008, Wiley and Sons Inc, Library of Congress, USA), Molecular Cloning: A Laboratory Manual, Third Edition, (Sambrook et al, 2001, Cold Spring Harbor, New York: Cold Spring Harbor Laboratory Press); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986). Antibodies : A Laboratory Manual, E. Howell and D Lane, Cold Spring Harbor Laboratory, 1988.

Par rapport aux complexes de ciblage de l'antigène de l'art antérieur qui ciblent une molécule de surface spécifique des cellules présentatrices de l'antigène (simple ciblage), le complexe selon l'invention permet un double ciblage de l'antigène sur une molécule de surface ubiquitaire, l'héparane sulfate, un sucre polysulfaté de la famille des glycosaminoglycanes, et sur une molécule de surface spécifique des cellules présentatrices de l'antigène. Le double ciblage de l'antigène sur les cellules présentatrices de l'antigène avec le complexe de l'invention augmente significativement la réponse immunitaire spécifique de l'antigène par rapport au simple ciblage avec les complexes de l'art antérieur. En outre, pour certains au moins des ligands, le double-ciblage avec le complexe de l'invention produit un effet synergique sur la réponse immunitaire spécifique de l'antigène par rapport au simple ciblage avec les complexes de l'art antérieur. Du fait de son immunogénicité accrue, le complexe de ciblage de l'antigène selon l'invention présente les avantages suivants par rapport aux complexes de l'art antérieur :
- il induit un effet protecteur plus important, permettant d'améliorer l'efficacité vaccinale.
- il permet de limiter le nombre d'administrations de rappel à effectuer.
- il permet de diminuer les doses injectées.
- il permet de s'affranchir de l'utilisation d'adjuvants de l'immunité.

Outre les dispositions qui précèdent, l'invention comprend encore d'autres dispositions qui ressortiront de la description qui va suivre, qui se réfère à des exemples de mise en oeuvre complexe objet de la présente invention ainsi qu'aux dessins annexés, dans lesquels :

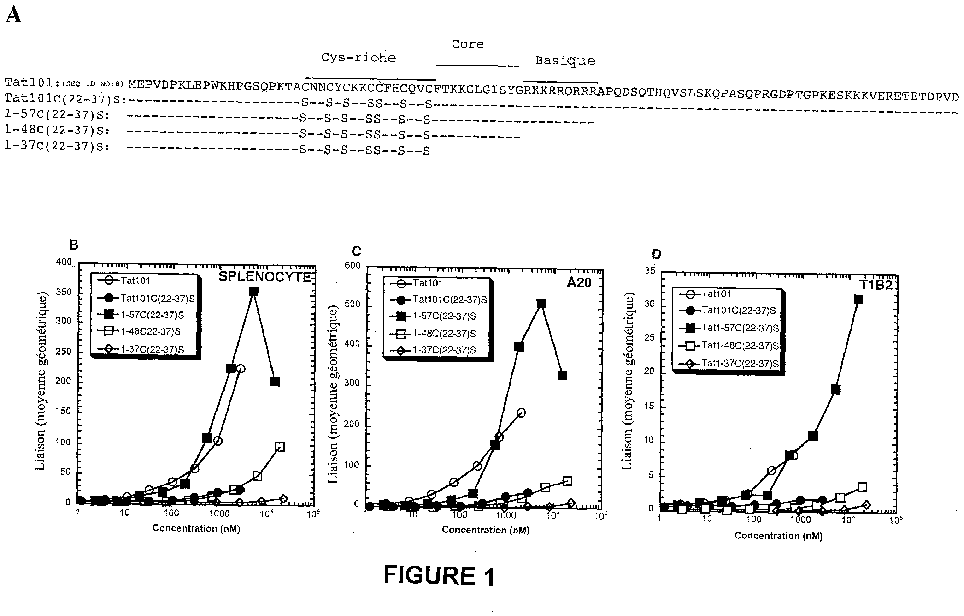

- la figure 1 illustre la liaison aux cellules de Tat101 et de quatre peptides dérivés de Tat. **A.** Séquence de Tat101, Tat101C(22-37)S et de trois peptides chevauchants. Les régions riche en cystéines (22-37), centrale (core : 38-48) et basique (49-57) sont soulignées. **B, C, D:** Liaison aux cellules de Tat101 et de quatre peptides dérivés de Tat. Des séries de dilutions des cinq peptides ont été incubées en présence de respectivement, des splénocytes **(B),** des cellules A20 de lymphome B, **(C)** et des cellules T1B2 d'hybridome T **(D).** Après 30 min d'incubation à 4 °C, un anticorps monoclonal anti-Tat couplé de façon covalente à la fluorescéine a été ajouté et la liaison des peptides aux cellules a été évaluée en cytométrie de flux (FACS). Des résultats similaires ont été obtenus dans deux expériences distinctes.

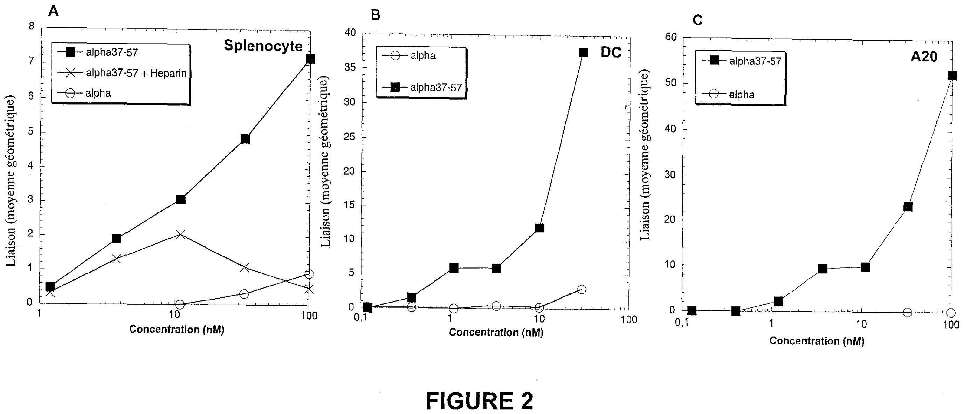

- la figure 2 illustre la liaison à différentes cellules présentatrices de l'antigène de la toxine alpha sous forme libre ou couplée au peptide 37-57 de Tat. **A.** Des splénocytes ont été incubés avec des séries de dilutions de la toxine alpha (alpha) ou alpha37-57, en présence ou en l'absence d'un excès d'héparine (3 µM). Après 30 minutes d'incubation à 4 °C, un anticorps polyclonal de lapin anti-toxine alpha a été ajouté. Ensuite, un fragment F(ab)'2 d'anticorps polyclonal de chèvre anti-IgG de lapin couplé à la fluorescéine a été ajouté pendant 30 minutes et la liaison de la toxine aux cellules a été analysée en FACS. **B.** Liaison aux cellules dendritiques (DCs), analysée selon le même protocole. C. Liaison aux cellules A20, analysée selon le même protocole. Des résultats similaires ont été obtenus dans aux moins deux expériences distinctes.

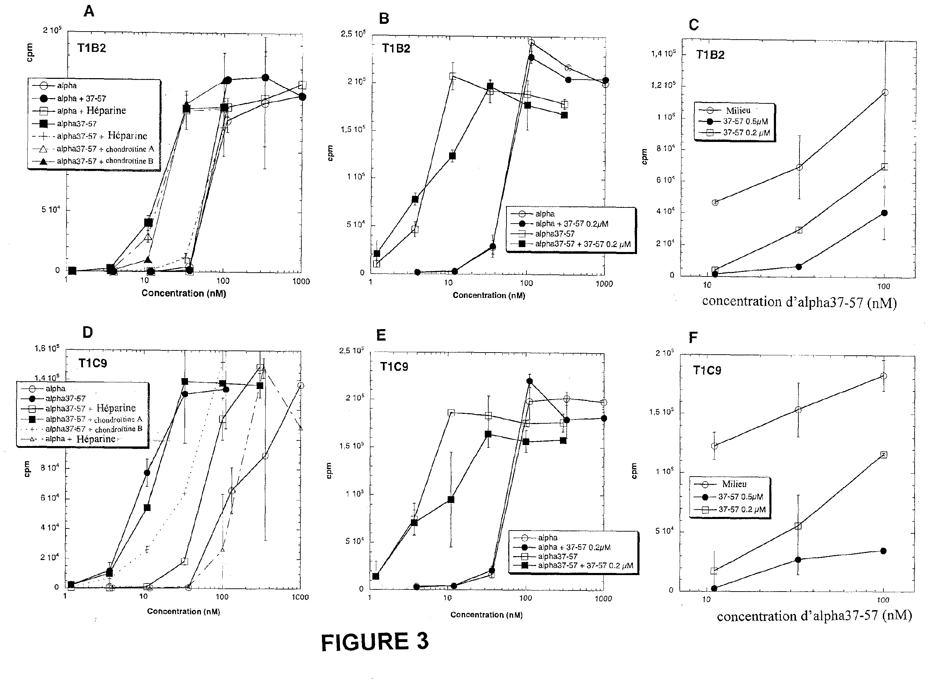

- la figure 3 montre que la capacité de la toxine alpha à stimuler les cellules T est augmentée quand elle est préalablement couplée au fragment 37-57 de Tat et que l'effet stimulant est altéré en présence d'un excès d'héparine ou de 37-57. Des splénocytes (5 10⁵/puits) ont été incubés avec des dilutions en série de toxine alpha, de toxine alpha plus peptide 37-57, ou d'alpha37-57, en présence ou en absence d'un excès (3 µM) de polysaccharides sulfatés (héparine, chondroïtine A, chondroïtine B) (**A** et **D**)**.** Après trois heures, les cellules T1B2 (**A**) ou T1C9 (**D**) ont été ajoutées et les mélanges ont été incubés pendant 24 heures. Le niveau de stimulation T qui se traduit par une sécrétion d'IL-2 a été évalué par un test de prolifération d'une lignée de CTL-IL-2-dépendante. Un protocole similaire a été utilisé pour examiner la présentation de l'antigène en présence ou en l'absence d'une quantité fixe (0,2 µM) de peptide libre 37-57 (**B** et **E**)**.** Une analyse supplémentaire de l'inhibition obtenue avec le peptide 37-57 est montrée en **C** et **F.** Dans ces expériences, les splénocytes (10⁵/puits) ont été pré-incubés à 4 °C pendant 30 minutes en présence ou en l'absence de peptide 37-57. Alpha37-57 a ensuite été ajouté aux puits pendant une heure à 4 °C. Les plaques ont ensuite été lavées, et les cellules T1B2 (**C**) ou T1C9 (**F**) ont été ajoutées pendant 24 heures à 37 °C. Le niveau de stimulation T qui se traduit par une sécrétion d'IL-2 a été évalué par un test de prolifération d'une lignée de CTL-IL-2-dépendante. Les résultats sont exprimés en cpm (nombre de coups par minute). Des résultats similaires ont été observés dans au moins deux expériences distinctes.

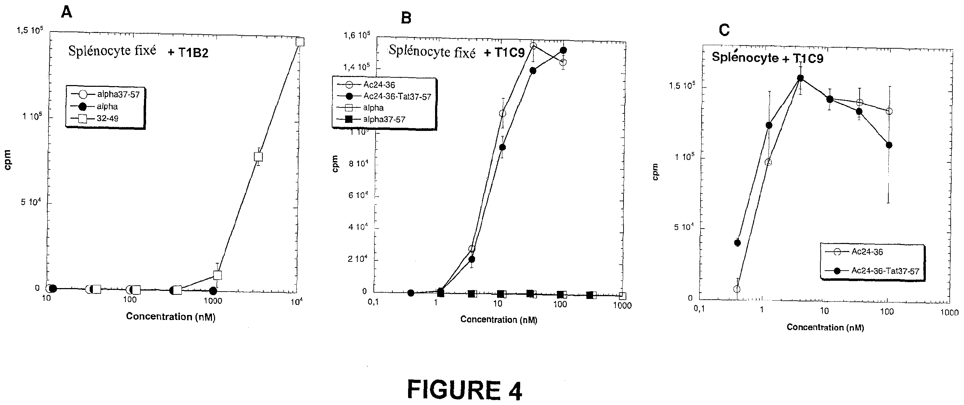

- la figure 4 montre que l'effet stimulant nécessite l'apprêtement de l'antigène. Pour examiner la nécessité d'apprêtement, des splénocytes préalablement fixés ont été incubées pendant trois heures, soit avec une série de dilutions de la toxine alpha, alpha37-57 ou de peptide 32-49 avant l'addition de T1B2 (**A**) ou avec une série de dilutions de toxine alpha, alpha37-57, de peptide Ac24-36 ou de peptide Ac24-36-Tat37-57 avant l'addition de T1C9 (**B**). Pour comparer les capacités stimulantes d'Ac24-36 et Ac24-36-Tat37-57 en présence de CPAgs vivantes, les splénocytes ont été incubés pendant 3 heures, avec ces deux peptides, respectivement (**C**). T1C9 a été ajoutée et la stimulation des cellules T a été évaluée comme décrit dans la figure 3. Des résultats similaires ont été observés dans au moins deux expériences distinctes.

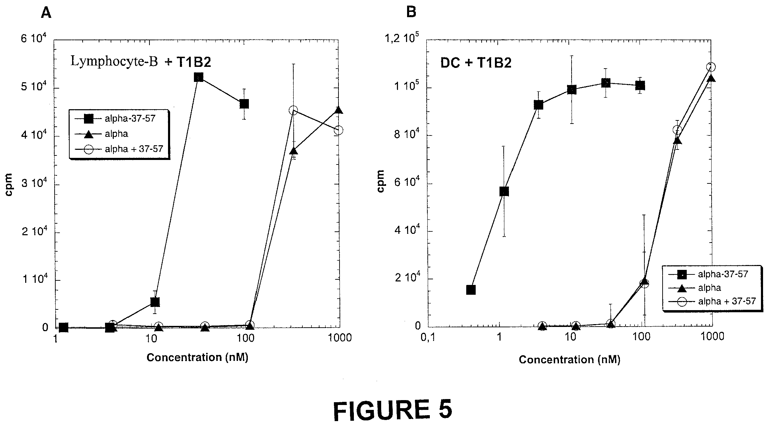

- la figure 5 montre que le fragment de Tat 37-57 augmente la présentation de la toxine alpha à T1B2 par les lymphocytes B et les DCs spléniques. Les lymphocytes B (**A**) ou les DCs (**B**) ont été incubés avec des dilutions en série de toxine alpha, toxine alpha plus peptide 37-57, ou alpha37-57. Après trois heures à 37 °C, les cellules T1B2 ont été ajoutées et la stimulation des cellules T a été évaluée comme décrit dans la figure 3. Des résultats similaires ont été observés dans au moins deux expériences distinctes.

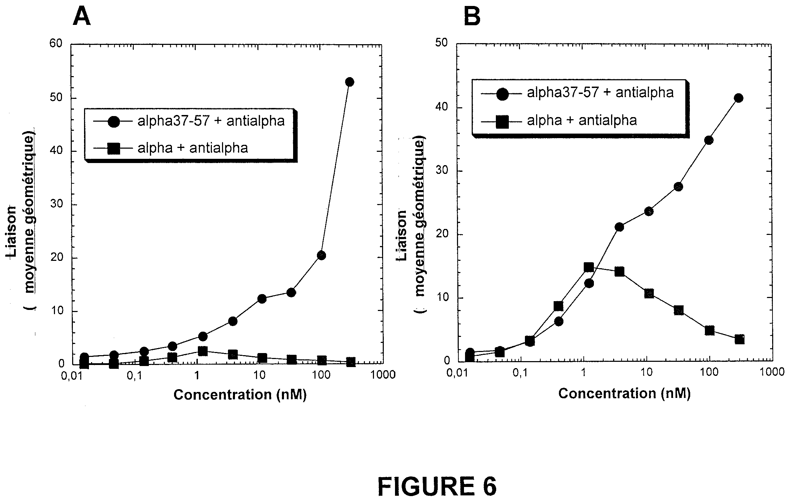

- la figure 6 montre que les complexes immuns contenant alpha37-57 se lient aux cellules A20 dépourvues de RFcγII ou exprimant le RFcγII et que le complexe alpha37-57 + antialpha lie le récepteur Fcy de type II de façon similaire à alpha + antialpha pour de faibles concentrations en antigène. Des dilutions en série d'alpha37-57 ont été incubées pendant une nuit en présence de quantités fixes d'un anticorps polyclonal de lapin antialpha (10 nM ou 25 nM). Les cellules A20 dépourvues de RFcγII (**A**) ou exprimant le RFcγII (**B**) ont ensuite été ajoutées aux mélanges et incubées pendant 30 minutes à 4 ° C. Les cellules ont ensuite été lavées et un fragment F(ab)'2 d'anticorps polyclonal de chèvre anti-IgG de lapin couplé à la fluorescéine a été ajouté afin d'évaluer la liaison des complexes immuns par analyse en FACS. Des résultats similaires ont été observés dans quatre expériences distinctes.

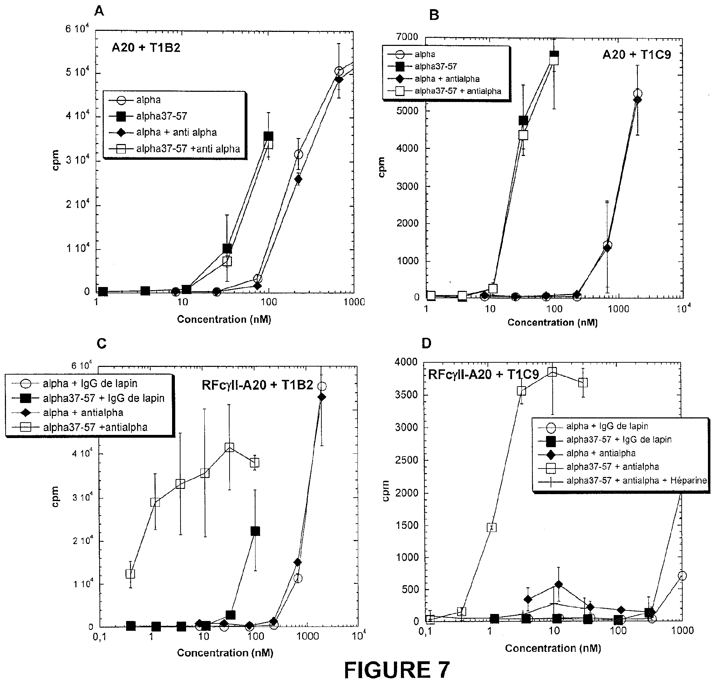

- la figure 7 montre que le complexe alpha37-57 + antialpha présente une capacité T-stimulante accrue. Des séries de dilutions de toxine alpha ou alpha-37-57 ont été pré-incubées une nuit à 4 °C en présence ou en absence d'une quantité fixe d'IgGs de lapin ou d'anticorps polyclonaux de lapin antialpha (12,5 nM). Les cellules A20 dépourvues de RFcγII (**A, B**) ou exprimant RFcγII (**C, D**) ont ensuite été ajoutées. Après 3 heures d'incubation à 37 °C, T1B2 (**A, C**) ou T1C9 (**B, D**) ont été ajoutés et incubés pendant 24 heures à 37 °C. La sécrétion d'IL-2 a été évaluée à l'aide d'une lignée dont la croissance dépend de la présence d'IL-2. Des résultats similaires ont été observés dans au moins deux expériences distinctes.

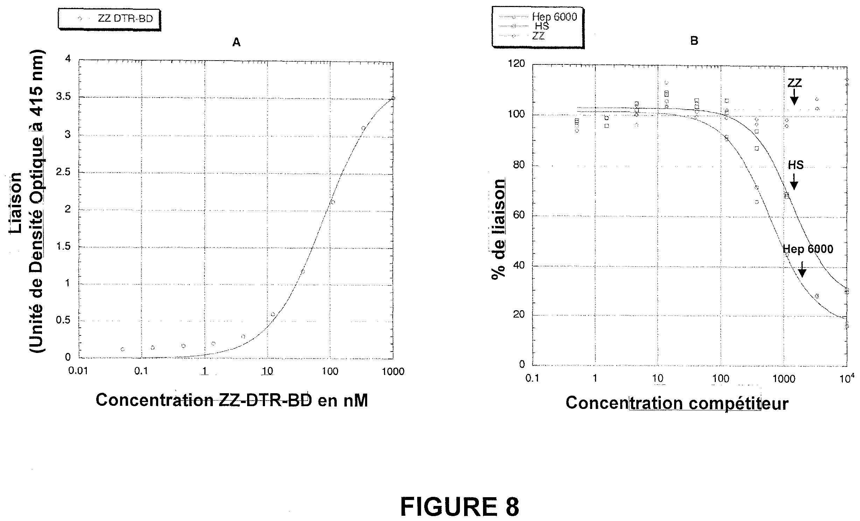

- la figure 8 montre que la protéine de fusion ZZDTR-BD est capable de lier l'héparine et l'héparane sulfate. Des séries de dilution de ZZDTR-BD ont été incubées dans des plaques de microtitration préalablement adsorbées avec de l'héparine couplée à de l'albumine pour évaluer la capacité de la protéine de fusion à lier le sucre sulfaté (**A**). La liaison aux plaques a été détectée à l'aide d'un anticorps polyclonal de lapin et d'un anticorps de chèvre anti-anticorps de lapin couplé à la péroxydase et d'un substrat de cet enzyme (ABTS). Pour évaluer la spécificité de liaison aux plaques et la région de ZZDTR-BD impliquée dans l'interaction, une quantité fixe de ZZDTR-BD a été incubée dans les plaques en présence de séries de dilutions de ZZ, Hep6000 et HS, respectivement (**B**). La liaison aux plaques a été détectée de la même manière qu'en A.

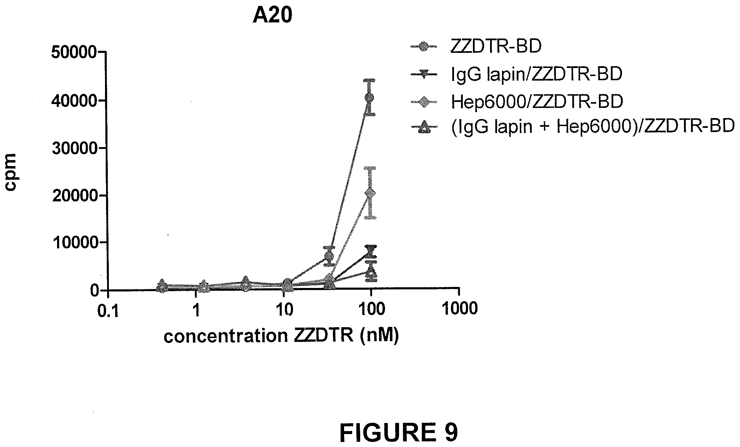

- la figure 9 montre que la capacité T-stimulante de ZZDTR-BD dépend de son aptitude à lier les HS et des immunoglobulines. ZZDTR-BD a été pré-incubé en présence ou en absence d'un excès d'Hep6000 (3µM), d'IgG de lapin (0,8µM), ou d'un mélange d'Hep6000 (3µM) et d'IgG de lapin (0,6 µM). Les mélanges ont été ajoutés aux cellules A20. Après 1 heure à 37 °C, l'hybridome T4B6 a été ajouté. 24 heures plus tard, les surnageants ont été prélevés et la présence d'IL-2 a été évaluée à l'aide d'une lignée CTL-IL-2 dépendante.

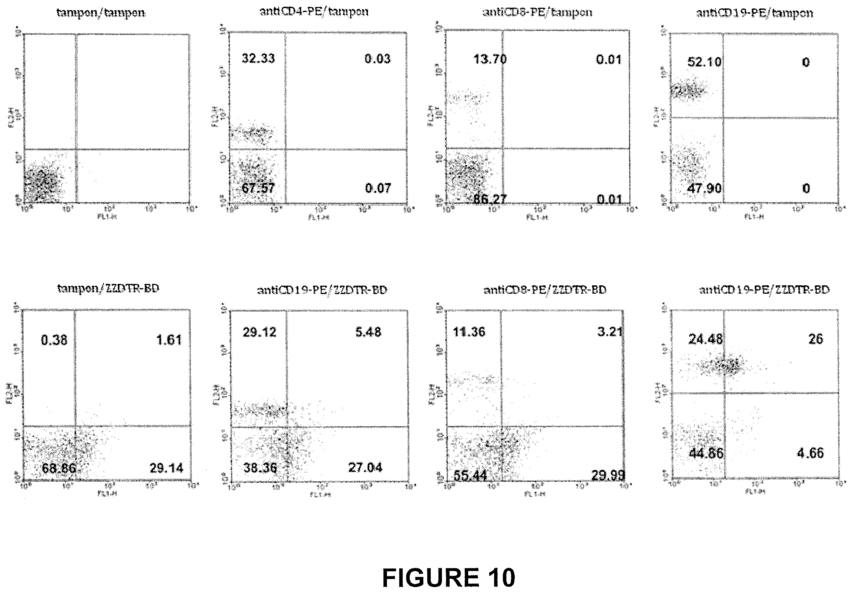

- la figure 10 illustre la détermination de la population de splénocytes liée par ZZDTR-BD. Une quantité fixe de ce complexe a été incubée en présence ou en absence de splénocytes et de trois anticorps marqués à la phycoerhytrine, spécifiques des lymphocytes T CD4+ (antiCD4-PE), des lymphocytes T CD8+ (antiCD8-PE) et des lymphocytes B (antiCD19-PE). Après 30 minutes à 4 °C, les cellules ont été lavées et incubées en présence d'un anticorps polyclonal anti-anticorps de lapin couplé à la fluorescéine. 30 minutes plus tard, les cellules ont été lavées et analysées par cytométrie de flux.

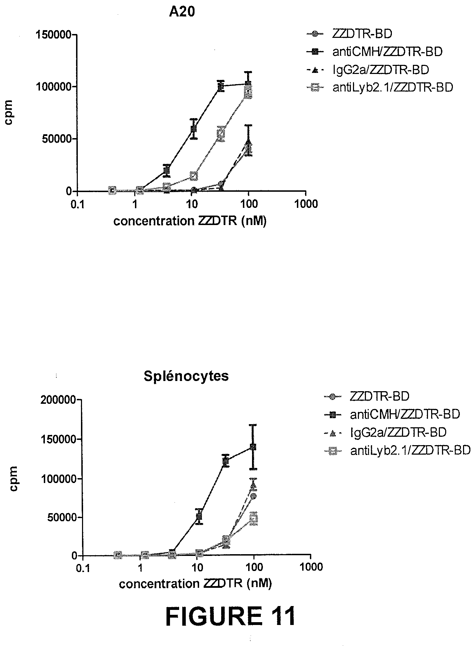

- la figure 11 montre que la capacité T-stimulante de ZZDTR-BD est augmentée quand elle est préalablement complexée à des anticorps spécifiques de déterminants exprimés à la surface de CPAgs. ZZDTR-BD a été pré-incubé en présence ou en absence de quantités équimolaires de l'anticorps 14-4-4S, 10-1.D.2 et Mα2-3, respectivement. Les mélanges ont été ajoutés à des cellules A20 ou à des splénocytes. Après 1 heure à 37 °C, l'hybridome T4B6 a été ajouté. 24 heures plus tard, les surnageants ont été prélevés et la présence d'IL-2 a été évaluée à l'aide d'une lignée CTL-IL-2 dépendante.

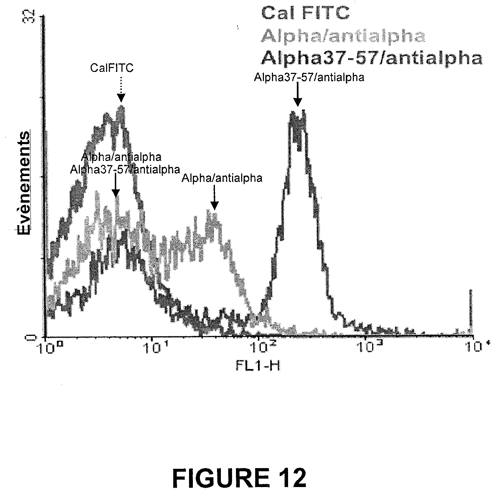

- la figure 12 illustre la liaison des complexes alpha/anti/alpha et alpha37-57/antialpha à des splénocytes de souris BALB/c, analysée en cytométrie de flux à l'aide d'un fragment F(ab)'2 d'anticorps polyclonal de chèvre anti-IgG de lapin couplé à la fluorescéine. Cal FITC : splénocytes (contrôle). Alpha/antialpha : splénocytes + complexes immuns alpha-antialpha. Alpha37-57/antialpha : splénocytes + complexes immuns alpha37-57/antialpha.

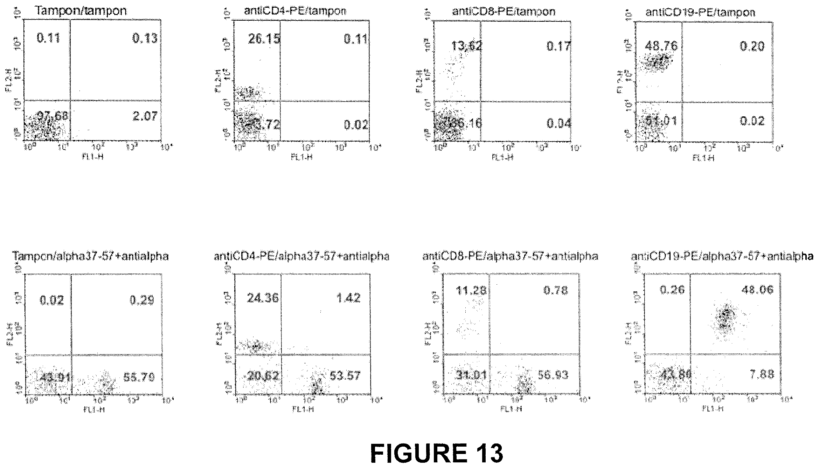

- la figure 13 illustre la détermination de la population de splénocytes liée par le complexe anti-toxine alpha/toxine alpha37-57. Une quantité fixe de ce complexe a été incubée en présence ou en absence de splénocytes et de trois anticorps marqués à la phycoerhytrine, spécifiques des lymphocytes T CD4+ (antiCD4-PE), des lymphocytes T CD8+ (antiCD8-PE) et des lymphocytes B (antiCD19-PE). Après 30 minutes à 4 °C, les cellules ont été lavées et incubées en présence d'un anticorps polyclonal anti-anticorps de lapin couplé à la fluorescéine. 30 minutes plus tard, les cellules ont été lavées et analysées par cytométrie de flux.

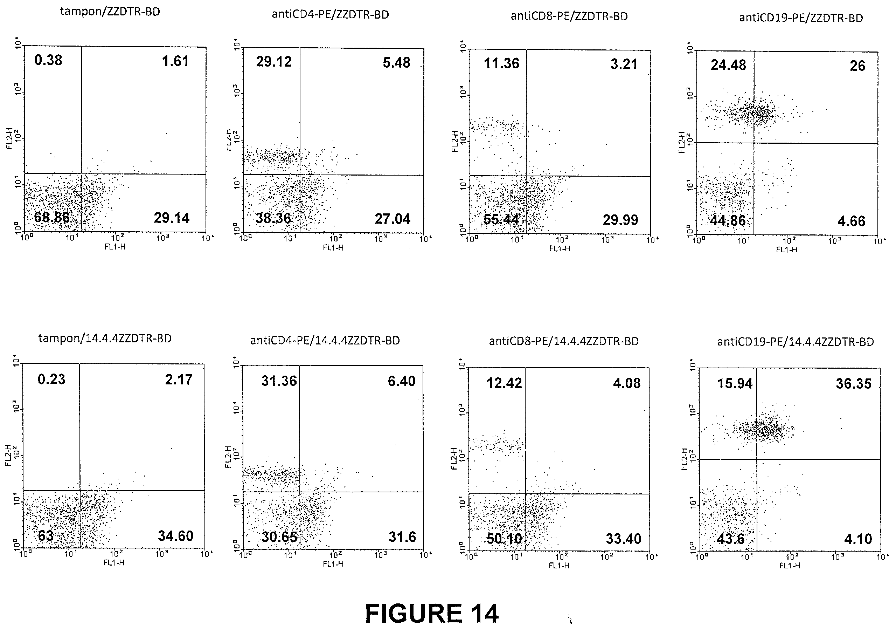

- la figure 14 illustre la détermination de la population de splénocytes liée par le complexe 14-4-4S/ZZDTR-BD. Une quantité fixe de ZZDTR-BD ou du complexe 14-4-4S/ZZDTR-BD a été incubée en présence ou en absence de splénocytes et de trois anticorps marqués à la fluorescéine, spécifiques des lymphocytes T CD4+ (antiCD4-PE), des lymphocytes T CD8+ (antiCD8-PE), et des lymphocytes B (antiCD19-PE). Après 30 minutes à 4 °C, les cellules ont été lavées et incubées en présence d'un anticorps polyclonal anti-anticorps de lapin couplé à la fluorescéine. 30 minutes plus tard, les cellules ont été lavées et analysées par cytométrie de flux.

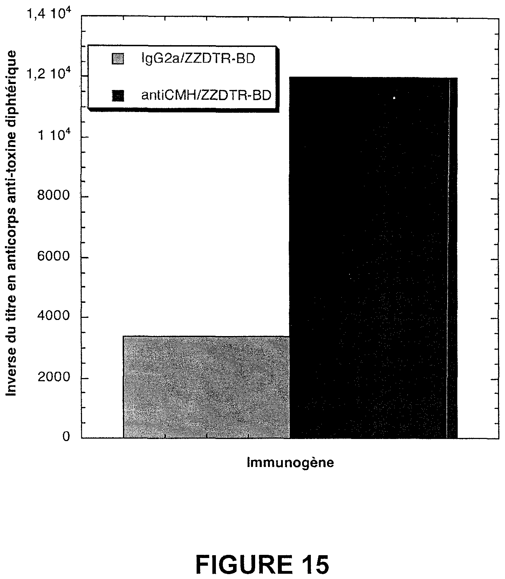

- la figure 15 montre que le complexe antiCMH/ZZDTR-BD induit en absence d'adjuvant une réponse en anticorps anti-toxine diphtérique supérieure à celle induite par le complexe IgG2a/ZZDTR-BD. Les deux complexes ont été respectivement injectés en absence d'adjuvant à deux groupes de 4 souris BALB/c. Les animaux ont été prélevés quarante cinq jours après l'immunisation et les sérums ont été poolés. La présence d'anticorps anti-toxine diphtérique a été évaluée par dosage immunoenzymatique.

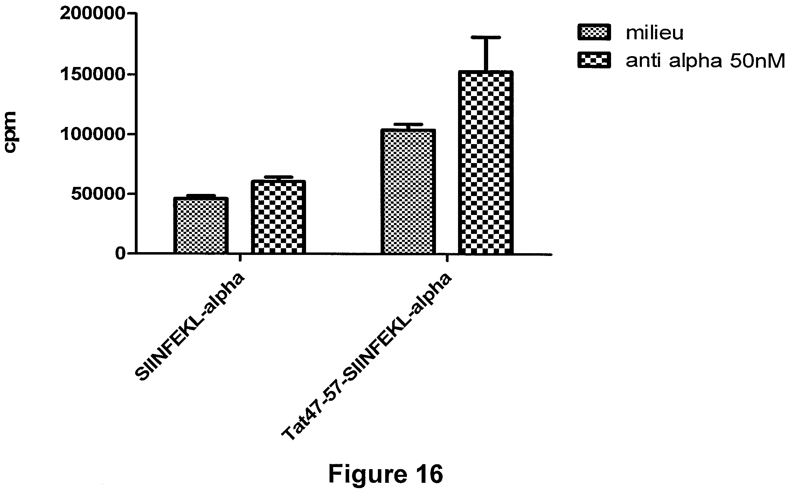

- la figure 16 montre qu'un composé possédant la capacité à cibler les HS et une molécule exprimée spécifiquement à la surface des CPAgs est capable d'induire une réponse immunitaire cytotoxique supérieure à un composé qui ne cible que les HS ou que la molécule exprimée spécifiquement à la surface des CPAgs. Les protéines Tat47-57-SIINFEKL-alpha et SIINFEKL-alpha (dilution 1µM final), complexées ou non complexées à l'anticorps polyclonal de lapin anti-toxine alpha (anti-alpha, 50nM) ont été ajoutés à des cellules dendritiques JAWSII utilisées comme CPAgs. Après 5 h à 37 °C, les cellules ont été fixées et des splénocytes de souris OTI qui contiennent des lymphocytes TCD8+ reconnaissant l'épitope T, de séquence SIINFEKL, en association avec les molécules de classe I de type I-A^{b}, ont été ajoutés. Le niveau de stimulation T CD8+ des splénocytes OTI a été évalué par un test de prolifération cellulaire. Les résultats sont exprimés en cpm (nombre de coups par minute).

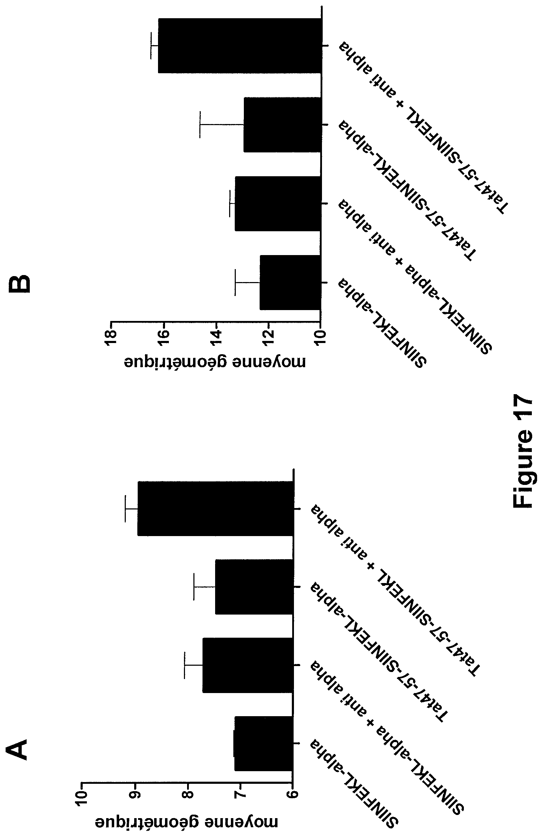

- la figure 17 montre qu'un composé possédant la capacité à cibler les HS et une molécule exprimée spécifiquement à la surface des CPAgs accroît plus fortement l'expression des molécules de co-stimulation CD80 et CD86 que les composés qui ne ciblent que les HS ou que la molécule exprimée spécifiquement à la surface des CPAgs. La toxine alpha sauvage et les protéines Tat47-57-SIINFEKL-alpha et SIINFEKL-alpha (1µM final) complexées ou non complexées à l'anticorps (25nM final) ont été ajoutées à des cellules dendritiques JAWSII utilisées comme CPAgs. Après 24 heures à 37°C, les cellules ont été lavées, incubées en présence d'anticorps anti-CD80 (A) et anti-CD86 (B) marqués à la fluorescéine, et la liaison des anticorps aux cellules a été analysée par cytométrie de flux.

### Exemple 1: Sélection d'un fragment de Tat capable de lier les héparanes sulfates de la surface cellulaire et couplage à la toxine alpha.

### 1. Matériels et méthodes

### 1.1 Synthèse des peptides Tat

La protéine Tat (SEQ ID NO : 8 ; Figure 1A) correspond à celle de l'isolat NDK du VIH-1 (Groenink et al., J. Virol., 1991, 65, 1968-1975) qui représente la séquence consensus obtenue à partir de 66 séquences d'isolats primaires de VIH-1 rapportées dans les bases de données SWISSPROT et TrEMBL entre 1999 et 2000. La synthèse chimique des peptides Tat a été réalisée par la stratégie Fmoc/tert-butyl à l'aide d'un synthétiseur automatique de peptides APPLIED BIOSYSTEMS 433A. Le procédé chimique utilise 0,1 mmole de résine Fmoc-Asp(OtBu)-PAL-PEG-PS, un excès d'un facteur 10 de chaque acide aminé, du dicyclohexylcarbodiimide/1-hydroxy-7-azabenzotriazole et du diisopropylethylamine/N-methyl pyrrolidone. Le clivage et la déprotection ont été réalisés à l'aide d'un mélange acide trifluoro-acetique/triisopropylsilane/eau (9,5/0,25/0,25, v/v/v). Le matériel brut a été précipité deux fois avec du ter-butyle méthyle éther refroidi 4 °C puis dissous dans une solution aqueuse d'acide acétique à 15 %. Les peptides ont ensuite été purifiés par chromatographie liquide haute performance (CLHP) en phase inverse, sur une colonne Vydac C18 (HESPERIA). Les peptides et les protéines synthétisés ont été caractérisés par spectrométrie de masse et par l'analyse des acides aminés. Ils sont conservés à 20 °C, sous forme lyophilisée.

### 1.2 Couplage du peptide Tat₃₇₋₅₇ à la toxine alpha

La toxine alpha de *Naja nigricollis* (Swiss-Prot P01468 ; SEQ ID NO : 9) a été purifiée comme décrit dans Fryklund et al., Biochemistry, 1975, 14, 2865-2871. La toxine alpha monothiolate, comprenant un groupement thiol en N-terminal, a été obtenue en utilisant le 3-(2- pyridyldithiol) propionate de N-succinimidyle (SPDP) comme réactif bifonctionnel, selon le protocole précédemment décrit (Léonetti et al., J. Exp. Med., 1999, 189, 12177-). Le pont disulfure supplémentaire a ensuite été réduit à l'aide d'un tampon acétate, pH 4,5, contenant 0,1 M NaCl et 25 mM dithiothreithiol. Le mélange a ensuite été agité à température ambiante, pendant 20 min et la solution a été filtrée sur une colonne PD10 équilibrée en tampon phosphate 0,1 M, pH 6,1, contenant 0,1 M NaCl. La toxine monothiolate éluée du volume mort a ensuite été incubée pendant 1 heure à température ambiante en présence d'un excès d'un facteur 3 du peptide Tat₃₇₋₅₇, dans du tampon PBS. Le mélange a ensuite été filtré sur une colonne PD10 équilibrée en tampon phosphate 0,1 M, pH 7 et la pureté du conjugué a été évaluée par chromatographie liquide haute performance (CLHP) en phase inverse.

### 1.3 Liaison aux cellules

### Cellules utilisées

La lignée de lymphome B murin, dénommée A20, obtenue comme décrit dans K.J. Kim *et al*., J. Immunol., 1979, 122, 549-, est disponible sous le n° ATCC TIB-208.

L'hybridome T, spécifique de la toxine α et de l'érabutoxine a, dénommé T1B2, a été obtenu comme décrit dans B. Maillère *et al*., J. Immunol., 1993, 150, 5270-.

Les splénocytes de souris ont été isolés à partir de rates de souris. Pour cela, les animaux ont été sacrifiés puis les rates ont été prélevées stérilement. Les rates ont été dilacérées et les globules rouges ont été lysés à 4°C à l'aide d'un tampon de Gey. Les splénocytes ont été récupérés après 10 minutes de centrifugation à 4°C.

Les cellules dendritiques ont été isolées à partir de splénocytes de souris en utilisant des microbilles MACS, selon le protocole du fabricant (MILTENYI BIOTEC). Brièvement, les rates ont été incubées en présence de collagénase D (2 mg/ml) pendant 30 minutes à 37 °C en présence d'un excès d'IgG de souris non-spécifiques. Les cellules ont ensuite été incubées à 4 °C. Quinze minutes plus tard, des microbilles antiCD11c ont été ajoutées et incubées pendant 15 minutes à 6-8 °C. Les splénocytes ont été lavés, centrifugés et passés au travers de colonnes magnétique. L'enrichissement a été évalué par analyse en FACS à l'aide de quatre anticorps anti-CD (antiCD4-FITC, anti-CD19-PE, anti-CD11c-FITC, et anti-IA/IE-PE, BECTON-DICKINSON). La pureté était supérieure à 95%.

### Liaison aux cellules de Tat101 et de peptides Tat avec des extensions C-terminales différentes

Des séries de dilutions de chaque peptide Tat ont été incubées en présence de différent types de cellules (2.10⁵ splénocytes par puits, 10⁵ cellules A20 ou T1B2 par puits) pendant 30 min à 4 °C en tampon PBS/0,5% BSA. Les cellules ont été lavées trois fois et incubées en présence d'anticorps monoclonal murin spécifique de la région N-terminale de Tat couplé de façon covalente à la fluorescéine tel que décrit dans Lecoq et al., Vaccine 2008, 26, 2615-2626 (1 µg/puits). Après 30 min d'incubation à 4 °C, les cellules ont été lavées et analysées en FACS.

### Liaison aux cellules de la toxine alpha sous forme libre ou couplée de façon covalente à Tat₃₇₋₅₇ (alpha37-57).

Des séries de dilutions de toxine alpha et alpha37-57 ont été incubées en présence de différent types de cellules (2.10⁵ splénocytes par puits, 10⁵ cellules A20 ou 10⁵ cellules dendritiques) pendant 30 min à 4 °C dans du tampon PBS/0,5% BSA. Les cellules ont été lavées et incubées en présence d'anticorps polyclonal de lapin anti-toxine alpha (1 µg/well). Après 30 min d'incubation à 4 °C, les cellules ont été lavées et un fragment F(ab)'2 d'anticorps polyclonal de chèvre anti-IgG de lapin couplé à la fluorescéine a été ajouté. Après 30 min d'incubation à 4 °C, les cellules ont été lavées et analysées en FACS®.

### 2. Résultats

Tat est une protéine liant l'héparine (Albini et al., Oncogene, 1996, 12, 289- ; Rusnati et al., J. Biol. Chem., 1997, 272, 11313-) qui est internalisée dans les cellules par un mécanisme nécessitant des héparanes sulfates protéoglycanes (HSPGs) à la surface des cellules (Tyagi et al., J. Biol. Chem., 2001, 276, 3254). Une protéine Tat sauvage de 101 résidus (Tat101) et quatre peptides dérivés de Tat ont été produits par synthèse chimique afin de sélectionner un fragment monomérique de Tat capable de lier les cellules (Figure 1A).

Dans ces dérivés, les sept résidus cystéine de Tat situés dans la région riche en cystéines de la molécule (acides aminés 22 à 37) ont été remplacés par sept sérines étant donné que Tat à une tendance naturelle à former une grande variété d'oligomères médiés par des ponts-disulfures (Kittiworakarn et al., J. Biol. Chem., 2006, 281, 3105-). Le premier dérivé est une molécule complète dénommée Tat101C(22-37)S (Kittiworakarn et al., J. Biol. Chem., 2006, 281, 3105-). Les trois autres polypeptides sont des fragments de Tat tronqués en C-terminal (1-57C(22-37)S, 1-48C(22-37)S, 1-37C(22-37)S). Après avoir évalué la pureté des cinq polypeptides, leur capacité à lier différents types de cellules a été comparée. Dans ces expériences, la liaison aux cellules a été révélée en utilisant un anticorps monoclonal dirigé contre la région N-terminale de Tat et une analyse en cytométrie de flux (FACS).

La figure 1B montre que Tat 101 lie les splénocytes alors que Tat101C(22-37)S interagit faiblement, indiquant que la région riche en cystéines joue un rôle important dans l'interaction de Tat avec les cellules. Contrairement à Tat101C(22-37)S, le peptide 1-57C(22-37)S lie les splénocytes comme Tat101 de type sauvage, indiquant que la perte de liaison due à l'absence des résidus cystéines a été contrebalancée par l'absence de la région 58-101. L'obtention d'une liaison similaire à celle du peptide 1-57C(22-37)S nécessite approximativement 50 fois plus de peptides 1-48C(22-37)S, indiquant que l'absence des résidus 49 à 57, correspondant à la région basique de Tat diminue fortement la capacité de liaison. Enfin, aucune liaison n'a été observée avec le peptide 1-37C(22-37)S, montrant que l'interaction est abolie en l'absence des résidus 38 à 48 correspondant au domaine central (core) de Tat.

Des résultats similaires ont été obtenus en utilisant les cellules de lymphome B murin de la lignée A20 (figure 1C) ou des cellules de l'hybridome T dénommé T1B2 (figure 1D), indiquant que Tat et ses fragments sont capables de lier une variété de cellules, en accord avec la distribution ubiquitaire des HSPGs. Du fait de ces résultats qui montrent l'implication des régions riche en cystéines (résidus 22-37), centrale (core : résidus 38 à 48) et riche en résidus basiques (résidus 49-57) dans la liaison aux cellules, et des observations antérieures que Tat a une propension spontanée à former une grande variété d'oligomères médiés par des ponts disulfures (Kittiworakarn et al., J. Biol. Chem., 2006, 281, 3105-), seules les régions riche en résidus basiques et centrale ont été sélectionnés pour préparer un fragment monomérique homogène capable de lier les cellules. La cystéine 37 de Tat a été incorporée dans ce fragment pour coupler la toxine alpha de *Naja nigricollis* (Thai et al., J. Biol. Chem., 2004, 279, 50257-). Le nouveau peptide synthétisé, dénommé 37-57 a été couplé à la toxine alpha via un pont disulfure entre la cystéine 37 et un groupement thiol préalablement incorporé sur la leucine 1 de la toxine alpha (Kessler et al., Bioconjug. Chem., 1994, 5, 199-), dans la mesure où ce résidu est éloigné des épitopes T de la toxine reconnus par l'haplotype H-2d (Léonetti et al., J. Immunol., 1990, 145, 4214- ; Maillère et al., J. Immunol., 1993, 150, 5270). Le dérivé a été dénommé alpha37-57.

Afin d'étudier si le peptide 37-57 confère à la toxine alpha la capacité de lier les cellules, différentes dilutions de toxine alpha ou de polypeptide alpha37-57 ont été incubées en présence de respectivement, des splénocytes, des cellules A20 ou des cellules dendritiques. Après 30 min d'incubation à 4 °C, un anticorps polyclonal de lapin anti-toxine alpha et un fragment F(ab)'2 d'anticorps polyclonal de chèvre anti-IgG de lapin couplé à la fluorescéine ont été ajoutés puis l'analyse par FACS a été effectuée.

La figure 2 montre que le polypeptide alpha37-57 augmente l'intensité de fluorescence de différents types de cellules alors que la toxine alpha libre (alpha) est peu efficace, suggérant fortement que le peptide 37-57 confère à la toxine alpha, la capacité de lier les cellules. Pour conforter le rôle de la portion 37-57 de Tat dans la liaison aux cellules, la liaison d'alpha37-57 aux cellules a aussi été examinée en présence d'héparine, un polysaccharide sulfaté soluble, représentatif des héparanes sulfates et capable de lier Tat (Albini et al., Oncogene, 1996, 12, 289). L'intensité de fluorescence diminue lorsque les splénocytes sont pré-incubés en présence d'alpha37-57 et d'un excès d'héparine (figure 2A), démontrant que la liaison d'alpha37-57 est médiée par la portion Tat.

### Exemple 2 : La toxine alpha est présentée de façon plus efficace à deux hybridomes de cellules T spécifiques de la toxine alpha lorsqu'elle est couplée de manière covalente au peptide 37-57.

### 1. Matériels et méthodes

### 1.1 Isolement de lymphocytes B et de cellules dendritiques à partir de splénocytes

Les cellules dendritiques ont été isolées comme décrit à l'exemple 1. Les lymphocytes B ont été isolés en utilisant des microbilles antiCD19, selon un protocole similaire.

### 1.2 Tests de stimulation des cellules T

Toutes les expériences ont été réalisées à l'aide de DCCM1 (BIOLOGICAL INDUSTRIES) comme milieu de culture synthétique. Des dilutions en série des différents antigènes ont été incubées dans les puits de micro-culture (NUNC) pendant 3 heures à 37 °C en présence, soit d'A20 (5.10⁴/puits), ou de splénocytes (5.10⁵/puits) ou de DCs (3.10⁴/puits). Un hybridome de cellules T spécifique de la toxine alpha (T1C9 : Maillère et al., Mol. Immunol., 1995, 32, 1073- ; T1B2 : Maillère et al., J. Immunol., 1993, 150, 5270- ; 5.10⁴/puits) a ensuite été ajouté aux puits et les cellules ont été cultivées pendant 24 h à 37 °C. Le niveau de stimulation T qui se traduit par une sécrétion d'IL-2 a été évalué par prélèvement des surnageants de culture et mesure de la présence d'IL-2 à l'aide d'une lignée de cellules T cytotoxiques (CTL) dont la croissance dépend de cette interleukine telle que décrite dans Gillis et al., Nature, 1977, 268, 154-156. La prolifération de la lignée CTL-IL-2 dépendante a été évaluée par mesure de l'incorporation de méthyl-thymidine tritiée (5 Ci/mmol). Les données sont exprimées en cpm.

### 2. Résultats

La présentation d'alpha37-57 et de la toxine alpha libre à deux hybridomes de cellules T spécifiques, dénommés T1B2 et T1C9, qui reconnaissent respectivement un épitope thiol-dépendant (Maillère et al., J. Immunol., 1993, 150, 5270-) et un épitope thiol-indépendant (Maillère et al., Mol. Immunol., 1995, 32, 1073-) a été évaluée en utilisant des splénocytes comme cellules présentatrices de l'antigène (APCs ou CPAgs). Comme le montre la figure 3A, la quantité de toxine alpha libre nécessaire pour stimuler T1B2 est similaire en présence et en l'absence de quantités équimolaires de peptide libre 37-57, ce qui indique que ce fragment de Tat libre n'a pas d'influence sur la présentation de l'antigène. En revanche, des quantités 5 fois inférieures d'alpha37-57 suffisent à stimuler les hybridomes de cellules T de manière aussi efficace que la toxine alpha libre, indiquant que l'augmentation de la capacité à stimuler les cellules T est due au couplage covalent du peptide Tat. Deux observations indiquent que l'effet stimulant est dû au ciblage de la protéine hybride sur les HSPGs exprimés à la surface des CPAgs. La première observation a été faite en utilisant trois polysaccharides solubles sulfatés différents incubées en excès (3 uM) avec des dilutions en série d'alpha et d'alpha37-57. Ni la chondroïtine A, ni la chondroïtine B qui sont des représentants des chondroïtines sulfates et qui lient faiblement Tat (Rustani et al., J. Biol. Chem., 1997, 272, 11313-), ne sont capables d'inhiber l'effet stimulant (figure 3A). En revanche, l'héparine, qui est représentative des héparanes sulfates et est capable d'inhiber la liaison de l'hybride alpha37-57 aux splénocytes (figure 2A), est capable de diminuer la capacité T-stimulante d'alpha37-57 au niveau observé avec la toxine alpha libre. La deuxième observation a été faite en utilisant des quantités en excès de peptide libre 37-57. Dans un première série d'expériences réalisées avec un protocole similaire à celui utilisé pour les polysaccharides sulfatés, le peptide 37-57 libre n'a pas d'incidence sur la présentation de la toxine alpha libre, indiquant qu'il n'est pas toxique à la concentration utilisée (0,2 µM) et qu'il inhibe faiblement, voire pas du tout, la présentation d'alpha37-57 (figure 3B). Toutefois, cette faible capacité d'inhibition est liée au protocole utilisé étant donné qu'une altération importante de la capacité T-stimulante a été observée lorsque l'étape de liaison aux cellules a été séparée de celle de l'apprêtement et de la présentation (figure 3C). Ainsi, la stimulation de l'hybridome de cellules T est diminuée lorsque les splénocytes sont incubés à 4 °C avec le peptide 37-57 et l'alpha37-57 et qu'ils sont ensuite lavés pour éliminer la protéine non liée avant l'addition des cellules T1B2. Ces observations ont également été faites lorsque la présentation de l'antigène a été étudiée au moyen de l'hybridome T1C9 (figure 3D, 3E, 3F). Ainsi, la protéine hybride est 13 fois plus puissante que la toxine alpha à stimuler T1C9, et ce phénomène est affecté en présence d'un excès d'héparine (figure 3D) ou de peptide libre 37-57 (figure 3F). Par conséquent, l'effet stimulant n'est pas lié à un épitope particulier. Comme la toxine alpha est une protéine très stable (Thai et al., J. Biol. Chem., 2004, 279, 50527-) qui nécessite absolument un apprêtement pour stimuler les cellules T (Maillère et al., J. Immunol., 1993, 150, 5270-), il a ensuite été examiné si l'effet stimulant dépendait de l'apprêtement de l'antigène. Pour ce faire, des splénocytes fixés ont été utilisés comme CPAgs. En leur présence, les deux hybridomes de cellules T ne sont pas stimulés par la toxine alpha, ni par alpha37-57 (figure 4A et 4B). Cette absence de stimulation n'est pas due à une altération de la capacité de présentation des splénocytes fixés, étant donné qu'ils restent capables de présenter le peptide 32-49 et le peptide Ac24-36 aux hybridomes T1B2 (figure 4A) et T1C9 (figure 4B), respectivement. Ces résultats indiquent donc que l'augmentation de la capacité T-stimulante dépend de l'apprêtement de la toxine par les CPAgs. Ensuite, il a été évalué si le fragment de Tat 37-57 pouvait également augmenter la présentation d'un antigène qui ne nécessite pas d'apprêtement. En comparant la capacité stimulante du peptide Ac24- -36 sous sa forme libre ou préalablement couplé à Tat37-57, il a été observé que Ac24-36 et Ac24-36-Tat37-57 ne diffèrent pas significativement dans leur capacité à stimuler T1C9, en présence de splénocytes fixés (figure 4B) ou vivants (figure 4C), indiquant que l'effet stimulant n'est pas lié à une augmentation de la présentation à la surface des cellules. L'ensemble de ces résultats indique que la fraction Tat37-57 cible les HSPGs et augmente ainsi la présentation restreinte au CMH de classe II d'un antigène protéique qui nécessite un apprêtement. Une augmentation similaire de la capacité T stimulante a été observée lorsque la toxine alpha est couplée à la région basique de Tat.

Ensuite, les lymphocytes et les DCs ont été purifiés à partir de splénocytes afin d'examiner si l'effet stimulant est observée en présence de ces deux types différents de CPAgs. Avec les lymphocytes B, la stimulation de T1B2 exige une quantité d'alpha37-57 environ 10 fois inférieure à celle de toxine alpha libre, ou de toxine alpha libre plus peptide 37-57 (figure 5A). Avec les DCs l'effet est plus prononcé, puisque la stimulation des cellules T nécessite 180 fois moins d'hybride que de toxine alpha libre en présence ou en l'absence de peptide 37-57 (figure 5B). Par conséquent, l'effet stimulant est observé en présence de ces deux types de CPAgs et dépend du couplage covalent du fragment de Tat à l'antigène protéique.

### Exemple 3 : Les CPAgs portant une récepteur Fcγ de type II (RFcγII) présentent de manière plus efficace un complexe immun contenant un antigène couplé de manière covalent au peptide 37-57.

### 1. Matériels et méthodes

### Cellules utilisées

### Voir l'exemple 1.

Les lignées de lymphome B, A20, dépourvues ou exprimant RFcγII, sont décrites dans Amigorena et al., Science, 1992, 256, 1808-.

### Liaison des complexes immuns aux cellules

Des séries de dilutions de toxine alpha ou alpha37-57 ont été incubées pendant une nuit à 4 °C, en présence d'un anticorps polyclonal de lapin anti-toxine alpha (10 nM ou 25 nM). Les cellules A20 et A20 exprimant RFcγII ont ensuite été ajoutées (10⁵ par puits) et incubées pendant 30 min à 4° C en tampon PBS complémenté avec 0,5 % de sérum albumine bovine (PBS/0,5% BSA). Les mélange ont ensuite été lavés et un anticorps de chèvre anti-IgG de lapin couplé à la fluorescéine été ajouté. Après 30 min d'incubation à 4 °C, les cellules ont été lavées et analysées par FACS®.

### Tests de stimulation des cellules T

### Voir l'exemple 2.

Des dilutions en série d'alpha ou d'alpha37-57 en présence ou en absence d'antialpha (12,5nM) ou d'anticorps de lapin non spécifiques de la toxine (appelés rabbit IgG) ont été pré-incubées une nuit à 4°C. Des cellules A20 dépourvues ou exprimant RFcγII (5 10⁴ cellules par puits) ont été ajoutées. Après 3 heures d'incubation à 37 °C, les hybridomes T1B2 et T1C9 ont été respectivement ajoutés à raison de 5.10⁴ cellules par puits. Après 24 heures de culture à 37 °C, le niveau de stimulation T qui se traduit par une sécrétion d'IL-2 a été évalué par prélèvement des surnageants de culture et mesure de la présence d'IL-2 à l'aide d'une lignée CTL dont la croissance dépend de cette interleukine. La prolifération de la lignée CTL-IL-2 dépendante a été évaluée par mesure de l'incorporation de méthyl-thymidine tritiée (5 Ci/mmol).

### 2. Résultats

Comme les HSPGs peuvent agir comme corécepteurs qui modulent la rencontre de protéines extracellulaires avec leurs récepteurs en formant des complexes héparanes sulfates (HS)/protéine (Park et al., J. Biol. Chem., 2000, 275, 29923- ; Carey, D.J. Biochem. J., 1997(Ptl) :1), alpha37-57 a été utilisée pour étudier si les HSPGs peuvent réguler la présentation de l'antigène médiée par un récepteur. La présente étude concerne la présentation médiée par le récepteur Fcy de type II (RFcγII) étant donné que ce récepteur lie les complexes immuns (ICs) et qu'il influence la présentation de l'antigène (Sallusto et al., J. Exp. Med., 1994, 179, 1109 ; Amigorena et al., Science, 1992, 256, 1808- ; Amigorena et al., J. Exp. Med., 1998, 187, 505- ; Regnault et al., J. Exp. Med., 1999, 189, 371-). En conséquence, un complexe immun a été utilisé. Ce complexe contient un anticorps polyclonal de lapin anti-toxine alpha (antialpha) et la toxine alpha de *Naja nigricollis* qui a préalablement été couplée au fragment 37-57 de Tat capable de lier les HS (alpha37-57). Ce complexe immun, appelé alpha37-57 + antialpha, possède donc la capacité à lier, d'une part, les HS exprimés à la surface de la plupart des cellules et, d'autre part, les récepteurs qui reconnaissent la région Fc des anticorps et qui sont sélectivement exprimés à la surface des CPAgs.

Ce complexe a été évalué en utilisant comme CPAgs, la lignée A20 de lymphome B, dépourvue ou exprimant le récepteur Fcγ de type II (RFcγII ; Amigorena et al., Science, 1992, 256, 1808-). Alpha37-57 + antialpha a été comparé à un complexe dépourvu de la région 37-57, appelé alpha + antialpha afin d'évaluer l'apport procuré par la région capable de lier les HS. Deux caractéristiques ont été étudiées : d'une part, la capacité à lier les deux types de cellules A20, d'autre part, la capacité à stimuler deux hybridomes T, appelés T1C9 et T1B2, qui sont spécifiques de la toxine alpha.

### 2.1. Capacité à lier les A20 dépourvues ou exprimant RFcγII : pour de faibles concentrations en antigène les complexes alpha37-57 + antialpha et alpha37-57 + antialpha lient de façon similaire le récepteur Fcγ de type II.

La liaison des deux complexes aux cellules A20 a été évaluée (figure 6). Comme on peut le voir sur la figure 6A, les cellules A20 qui n'expriment pas le RFcγII sont liées uniquement par le complexe alpha37-57 + antialpha ce qui indique que l'interaction du complexe est médiée par la région 37-57 de liaison aux HS. Le scénario est différent en présence des cellules A20 exprimant RFcγII (figure 6B). En effet, le complexe alpha + antialpha se lie aux cellules de façon similaire à alpha37-57 + antialpha pour des concentrations d'Ag inférieures ou égales à 1 nM ce qui indique que, pour de faibles concentrations en antigène, les deux complexes lient aussi bien les cellules. Ces observations indiquent donc que les deux complexes interagissent de façon similaire avec le RFcγII exprimé à la surface des cellules A20 ce qui indique que la présence du ligand des HS ne perturbe pas l'interaction des anticorps au RFcγII. Quand la quantité d'antigène devient supérieure à 1 nM l'interaction d'alpha + antialpha décroît ce qui indique que les anticorps lient moins efficacement les RFcγII exprimés à la surface des cellules. En revanche, la liaison d'alpha37-57 + antialpha augmente ce qui indique que l'interaction avec les cellules devient principalement médiée par la région 37-57.

### 2.2. Capacité T-stimulante : la capacité T-stimulante de la toxine alpha est augmentée quand la protéine est couplée à 37-57 et complexée à l'anticorps antialpha.

La capacité T-stimulante des complexes alpha+antialpha et alpha37-57+antialpha a été évaluée (figure 7). Comme on peut le voir sur cette figure, les cellules A20 négatives (figure 7A) et positives (figure 7C) pour RFcγII présentent mieux alpha37-57 qu'alpha à l'hybridome T1B2 ce qui indique que la présence du ligand des HS accroît la présentation de la protéine et sa capacité T-stimulante. En présence des A20 dépourvues de RFcγII, la présentation du complexe immun alpha37-57 + antialpha reste semblable à celle d'alpha37-57 libre et la présentation du complexe alpha + antialpha reste semblable à celle d'alpha (figure 7A et 7B) ce qui indique que la capacité T-stimulante de l'antigène (Ag) n'est pas modifiée lorsqu'il est associé à l'anticorps et présenté par ces CPAgs. Le résultat est similaire pour le complexe alpha + antialpha en présence des A20 exprimant RFcγII (figure 7C et 7D) ce qui indique que la capacité T-stimulante de la toxine alpha sauvage n'est pas modifiée lorsqu'elle est associée à l'anticorps qui interagit avec le récepteur Fc exprimé à la surface des CPAgs. Le résultat est en revanche sensiblement différent pour le complexe alpha37-57 + antialpha. En effet, la capacité T-stimulante d'alpha37-57 est fortement accrue lorsqu'il interagit préalablement avec l'antialpha (figure 7C et 7D). Ainsi, pour stimuler T1B2 avec alpha37-57 + antialpha il suffit de quantités d'Ag respectivement 130 fois plus faibles qu'alpha37-57 libre (alpha + rabbit IgG), et plus de 1000 fois inférieures à alpha libre (alpha + rabbitIgG) ou complexé à l'antialpha (alpha + antialpha). Le même phénomène est observé pour l'hybridome T1C9 ce qui indique que l'augmentation de capacité T-stimulante est procurée pour l'ensemble des épitopes T de la toxine alpha. Ces résultats indiquent donc que le ciblage du RFcγII permet un accroissement de la capacité T-stimulante de la toxine alpha complexée à l'anticorps seulement dans le cas où elle est préalablement couplée au fragment 37-57. De plus, l'augmentation du pouvoir T-stimulant disparaît lorsque le complexe est incubé en présence d'un excès d'héparine (voir alpha37-57 + antialpha + Héparine, figure 7D) ce qui indique que le fragment 37-57 contribue à l'effet.

L'ensemble de ces observations indique donc que la capacité T-stimulante de la toxine alpha est augmentée quand elle est associée à un ligand des HS et à un ligand du récepteur FcγII et que ce phénomène est provoqué par le ciblage du RFcγII en association avec la capacité à lier les HS.

### Exemple 4 : Le domaine de la toxine diphtérique capable de se lier au récepteur cellulaire est capable de lier les héparanes sulfates.

Des protéines de fusion incorporant, d'une part, une protéine antigénique et, d'autre part, un double domaine ZZ dérivé de la protéine A de *Staphylococcus aureus* ont été précédemment construites (Léonetti et al., J. Immunol., 1998, 160, 3820-). ZZ peut se lier aux immunoglobulines de façon semblable à la protéine A et il a été observé que des protéines de fusion de type ZZAg présentent une capacité T-stimulante accrue. Il a été montré que cet accroissement est médié par la région ZZ qui lie les CPAgs porteuses d'immunoglobulines et accroît ainsi la quantité d'Ag incorporée dans les cellules. Ce système de protéine de fusion permet donc un ciblage des Ags médié par les immunoglobulines de surface. Sur la base de ce système, une autre protéine de fusion, appelée ZZDTR dans la publication de Lobeck et al. (Infection and Immunity, 1998, 66, 418-423), a été construite. Cette protéine de fusion, appelée maintenant ZZDTR-BD, contient le domaine (DTR-BD) de la toxine diphtérique capable de lier le récepteur cellulaire qui est la cible de cette toxine. Ce récepteur n'est pas exprimé à la surface des CPAgs. Il a été évalué si la protéine de fusion ZZDTR-BD était capable de lier les héparanes sulfates.

La liaison de ZZDTR-BD aux héparanes sulfates a été évaluée en deux étapes.

Dans une première étape, la liaison de ZZDTR-BD à des plaques de microtitration portant de l'héparine a été évaluée. Pour cela, les plaques de microtitration ont été préalablement adsorbées avec de l'héparine-albumine (1µg/100µl/puits) puis saturées avec de la sérum albumine bovine (200µl/puits à 0,3 %). Les plaques ont ensuite été lavées et une série de dilutions de ZZDTR-BD a été déposée dans les puits et incubée pendant une nuit à 4°C. Les plaques ont été lavées et un anticorps polyclonal de lapin a été ajouté. Après 30 minutes à température ambiante, les plaques ont été lavées et un anticorps de chèvre anti-anticorps de lapin a été ajouté. 30 minutes plus tard, les plaques ont été lavées, un substrat a été ajouté et la coloration a été mesurée à 414 nm après 30 minutes d'incubation.

Dans une seconde étape, il a été évalué si la liaison de ZZDTR-BD aux plaques adsorbées avec l'héparine-albumine peut être altérée lorsque la protéine de fusion est incubée en présence de solutions contenant soit un fragment d'héparine de 6000Da de masse moléculaire (Hep6000), soit de l'héparane sulfate soit ZZ. Pour cela, une concentration fixe de ZZDTR-BD a été déposée en présence de séries de dilutions d'Hep6000, d'héparane sulfate (HS) ou de ZZ. Après une incubation d'une nuit à 4° C, les plaques ont été lavées et un anticorps polyclonal de lapin a été ajouté. Après 30 minutes à température ambiante, les plaques ont été lavées et un anticorps de chèvre anti-anticorps de lapin a été ajouté. 30 minutes plus tard, les plaques ont été lavées, un substrat a été ajouté et la coloration a été mesurée à 414nm après 30 minutes d'incubation.

Comme on peut le voir sur la figure 8A, ZZDTR-BD lie les plaques adsorbées avec l'héparine-albumine ce qui indique que la protéine de fusion est capable d'interagir avec ce sucre sulfaté qui est proche des héparanes sulfates. L'interaction de ZZDTR-BD aux plaques est inhibée par les solutions d'Hep6000 et d'HS ce qui indique que la protéine de fusion interagit avec les HS et l'héparine à partir d'un site commun. Or, ce site ne se trouve pas localisé dans la région ZZ car le double domaine ZZ libre n'altère pas la liaison de ZZDTR-BD aux plaques. On peut donc en conclure que la capacité de liaison aux HS est médiée par le domaine DTR-BD de ZZDTR-BD.

### Exemple 5: La protéine de fusion ZZDTR-BD présente une capacité T-stimulante qui est influencée par son aptitude à lier les HS et des immunoglobulines exprimées à la surface de CPAgs

Comme la protéine de fusion est capable de lier les HS et des immunoglobulines exprimées à la surface de CPAgs, il a été évalué si sa capacité T-stimulante dépend de ces deux caractéristiques. Pour cela, la présentation de ZZDTR-BD à un hybridome T spécifique de la toxine diphtérique a été étudiée « *in vitro ».* L'hybridome, appelé T4B6, reconnait l'épitope T 92-106 situé dans le domaine DTR-BD de la toxine diphtérique.

Pour évaluer la capacité T-stimulante, des séries de dilutions de ZZDTR-BD en présence ou en absence d'un excès d'Hep6000 (3µM final), d'HS (3µM final) ou d'IgG de lapin (0,8µM final) ont été incubées pendant 3 h à 4 °C dans des plaques de culture cellulaire. La lignée A20 qui est un lymphome B possédant des immunoglobulines en surface (5 10⁴ cellules/pts, figure 9) et doté de capacités de présentation a ensuite été rajoutée. Après 1 heure d'incubation à 37°C, l'hybridome T4B6 est ajouté et le mélange est incubé pendant 24 heures à 37°C. La stimulation de l'hybridome est ensuite évaluée par mesure de la présence d'IL-2 dans les surnageants. Pour cela, les surnageants sont prélevés et incubés en présence d'une lignée CTL dont la pousse dépend de la présence d'IL-2. La croissance cellulaire est évaluée par mesure de l'incorporation de thymidine tritiée dans la lignée CTL-IL-2 dépendante.

Comme on peut le voir sur la figure 9, en présence du lymphome B A20, ZZDTR-BD est capable de stimuler l'hybridome T4B6 spécifique de la toxine diphtérique. La stimulation est plus faible lorsque ZZDTR-BD est préalablement incubé en présence d'un excès d'Hep6000 ce qui démontre que la capacité T-stimulante de la protéine est influencée par sa capacité à lier l'héparine. Comme il a précédemment été montré que le système de protéine de fusion ZZAg permet un ciblage des Ags vers les immunoglobulines exprimées à la surface des CPAgs (Léonetti et al., J. Immunol., 1998, 160, 3820-), ces résultats démontrent que ZZDTR-BD présente une capacité T-stimulante qui est accrue par sa capacité à lier des immunoglobulines et des composés de la famille des héparanes sulfates.

Pour déterminer la population de splénocytes liée par ZZDTR-BD, une quantité fixe de ZZDTR-BD (10 nM) a été incubée en présence de splénocytes et de trois anticorps respectivement spécifiques des lymphocytes T CD4+ (antiCD4), des lymphocytes T CD8+ (antiCD8) et des lymphocytes B (antiCD19). Ces trois anticorps sont marqués à la phycoerhytrine. Après 30 minutes à 4°C, les cellules ont été lavées et incubées en présence d'un anticorps polyclonal de lapin qui peut interagir avec la région ZZ de la protéine de fusion. 30 minutes plus tard, un anticorps polyclonal anti-anticorps de lapin couplé à la fluorescéine a été ajouté. Après 30 minutes d'incubation à 4°C, les cellules ont été lavées et analysées par cytométrie de flux. Comme on peut le voir sur la figure 10, les splénocytes contiennent environ 14 % de lymphocytes T CD8+, et 22 % de ces cellules sont liées par ZZDTR-BD. Les splénocytes contiennent environ 32 % de lymphocytes T CD4+, et 15,8 % de ces cellules sont liées par ZZDTR-BD. Les splénocytes contiennent environ 52 % de lymphocytes B, et 51,5 % de ces cellules sont liée par ZZDTR-BD. Comme les cellules B expriment des anticorps de surface et les cellules T CD4+ et T CD8+ en sont dépourvues, on peut conclure que ZZDTR-BD cible les anticorps de surface et ainsi se lie préférentiellement aux lymphocytes B, qui sont des CPAgs.

### Exemple 6: Augmentation de la capacité T-stimulante de la protéine de fusion ZZDTR-BD préalablement associée à des anticorps spécifiques de déterminants de surface de CPAgs.

La protéine A et le domaine Z qui en est dérivé peuvent se lier à la région Fc de différentes classes d'anticorps. Il a précédemment été montré que cette caractéristique peut être exploitée pour former des complexes entre des Acs et des protéines de fusion de type ZZAg (Léonetti et al., J. Immunol., 1998, 160, 3820-). Il a de plus été montré qu'un complexe Ac/ZZAg contenant un Ac spécifique d'un déterminant de surface de CPAg est mieux présenté aux cellules T que ZZAg libre ou complexé à un anticorps non-spécifique et qu'il est capable d'induire, chez l'animal, une réponse immunitaire en absence d'adjuvant. Le même principe de formation de complexes entre ZZDTR et des anticorps a donc été utilisé pour évaluer si la protéine de fusion qui possède la capacité à lier les HS peut ainsi voir sa capacité T-stimulante accrue lorsqu'elle interagit avec des anticorps capables de cibler des déterminants de surface de CPAgs.

Pour réaliser cette étude, trois anticorps monoclonaux de souris, de sous classe IgG2a, qui possèdent tous la capacité à lier ZZ, ont été sélectionnés. Le premier, appelé Mα2-3, décrit dans Trémeau et al., FEBS Lett., 1986, 208, 236-240, est utilisé à titre de contrôle car il ne lie pas les CPAgs. Les deux autres anticorps sont spécifiques de déterminants de surface de CPAgs. Le premier, appelé 14-4-4S, reconnaît la molécule I-E^{d} du CMH de classe II (Ozato et al., J. Immunol., 1980, 122, 549). Le second, appelé 10-1.D.2, reconnaît l'antigène Lyb-2.1 qui est exprimé à la surface des lymphocytes B (numéro ATCC TIB-165).

Pour évaluer la capacité T-stimulante de ZZ-DTR en présence d'anticorps, ZZDTR est dilué en présence ou absence de 14-4-4S, 10-1.D.2, Mα2-3, respectivement. Les anticorps sont incubés à 50 nM final. Suite à ces dilutions, les composés sont incubés 3h à 4 °C puis des CPAgs sont ajoutées. Les CPAgs sont, d'une part, des cellules A20 (5 10⁴/50µl/puits), d'autre part, des splénocytes (5 10⁵/50µl/puits) de souris BALB/c. Après 2 h à 37°C, l'hybridome T4B6 (5 10⁴/50µl/puits) est ajouté et le mélange est incubé 24 h à 37°C. La stimulation de la cellule T4B6 est ensuite évaluée par mesure de sa sécrétion d'interleukine 2. Cette mesure est réalisée à partir des surnageants de culture qui sont incubés sur une lignée CTL dont la pousse dépend la présence d'IL-2.

Comme on peut le voir sur la figure 11, en présence de cellules A20, ZZDTR-BD stimule T4B6. Des quantités de ZZDTR-BD approximativement 13,4 fois et 3, 8 fois inférieures sont requises pour atteindre le même niveau de stimulation lorsque la protéine de fusion est respectivement complexée à l'antiCMH (antiCMH/ZZDTR-BD) et à l'anti-Lyb-2.1 (antiLyb-2.1/ZZDTR-BD). L'accroissement de la capacité T-stimulante n'est pas dû au fait que l'antiCMH et l'antiLyb-2.1 soit des immunoglobulines de sous-classe 2a car l'effet n'est pas retrouvé lorsque ZZDTR-BD est complexé à une IgG2a non spécifique d'un déterminant de surface de CPAgs (IgG2a/ZZDTR-BD). Ces résultats démontrent donc que la capacité T-stimulante de ZZDTR-BD est accrue par ciblage des molécules de CMH de classe II ou Lyb-2.1 qui sont exprimées sélectivement à la surface des cellules A20 utilisées comme CPAgs.

En présence de splénocytes, ZZDTR-BD stimule T4B6. Des quantités de ZZDTR-BD approximativement 5,2 fois inférieures sont requises pour atteindre le même niveau de stimulation lorsque la protéine de fusion est complexée à l'antiCMH (antiCMH/ZZDTR-BD). En revanche quand ZZDTR-BD est complexé à l'anticorps anti-Lyb-2.1 (antiLyb-2.1/ZZDTR-BD) la capacité T-stimulante est légèrement diminuée. L'accroissement de la capacité T-stimulante médié par l'antiCMH ainsi que la diminution de capacité T-stimulante médié par l'antiLyb-2.1 ne sont pas dus au fait que ces immunoglobulines soient de sous-classe 2a car l'effet n'est pas retrouvé lorsque ZZDTR-BD est complexé à une IgG2a non spécifique d'un déterminant de surface de CPAgs (IgG2a/ZZDTR-BD). Ces résultats démontrent donc que la capacité T-stimulante de ZZDTR-BD est accrue par ciblage des molécules de CMH de classe II qui sont exprimées sélectivement à la surface des splénocytes.

### Exemple 7 : Deux Ags différents qui sont respectivement associés à un ligand des HS et à un anticorps capable de lier une protéine de surface des CPAgs lient préférentiellement et de façon accrue les CPAgs exprimant cette protéine de surface.

Deux expériences de liaison à des splénocytes ont été réalisées pour évaluer si les Ags qui sont associés à un ligand des HS et à un anticorps spécifique d'une protéine de surface des CPAgs sont capables de cibler préférentiellement des CPAgs.

### 7.1. Etude de liaison aux splénocytes à l'aide du système protéique décrit dans l'exemple 3

### 7.1.1. Etude de la liaison en présence ou en absence du ligand des héparanes sulfates.

La toxine alpha sauvage et la toxine alpha37-57 ont été préalablement incubées en présence ou en absence de l'anticorps polyclonal de lapin anti-toxine alpha. Les mélanges ont ensuite été ajoutés à des splénocytes (5 10⁵/100µl/puits) et incubés 30 minutes à 4 °C. Les cellules ont été lavées et incubées en présence d'un anticorps polyclonal anti-anticorps de lapin couplé à la fluorescéine. Après 30 minutes à 4 °C, les cellules ont été lavées et la liaison des complexes anti-toxine alpha/toxine alpha et anti-toxine alpha/toxine alpha37-57 a été évaluée par cytométrie de flux.

Comme on peut le voir sur la figure 12, les cellules incubées en présence de l'anticorps polyclonal anti-anticorps de lapin couplé à la fluorescéine présentent un faible niveau de fluorescence qui se traduit par une moyenne géométrique de 3,6. Les splénocytes préalablement incubés avec le complexe alpha/antialpha présentent un niveau de fluorescence moyen qui est accru d'un facteur 2,75 (moyenne géométrique de 9,9). Enfin, les cellules pré-incubées avec le complexe alpha37-57/antialpha présentent un profil mixte. Une première population de cellule présente un faible marquage (moyenne géométrique de 4,44) qui est peu différent de celui des cellules incubées en absence de complexe. Une deuxième population fluoresce en revanche beaucoup plus fortement (moyenne géométrique de 232,1, intensité multipliée 64,5). Ces observations indiquent donc que le complexe alpha37-57/antialpha présente la particularité de se lier de façon accrue à une sous population de splénocytes.

### 7.1.2. Détermination des sous-populations de splénocytes liées par le complexe anti-toxine alpha/toxine alpha37-57.

Pour déterminer la population de splénocytes liée par le complexe anti-toxine alpha/toxine alpha37-57, une quantité fixe de ce complexe (10 nM) a été incubée en présence ou en absence de splénocytes et de trois anticorps respectivement spécifiques des lymphocytes T CD4+ (antiCD4), des lymphocytes T CD8+ (antiCD8) et des lymphocytes B (antiCD19). Ces trois anticorps sont marqués à la phycoerhytrine. Après 30 minutes à 4 °C, les cellules ont été lavées et incubées en présence d'un anticorps polyclonal anti-anticorps de lapin couplé à la fluorescéine. 30 minutes plus tard, les cellules ont été lavées et analysées par cytométrie de flux.

Comme on peut le voir sur la figure 13, les splénocytes contiennent environ 14 % de lymphocytes T CD8+, et 6,4% de ces cellules sont liées par le complexe alpha37-57+antialpha. Les splénocytes contiennent environ 26 % de lymphocytes T CD4+, et 5,5% de ces cellules sont liées par le complexe. Les splénocytes contiennent environ 49 % de lymphocytes B et la quasi totalité de ces cellules est liée par le complexe. Comme les cellules B portent des récepteurs Fc alors que les cellules T CD4+ et T CD8+ en sont dépourvues, on peut donc conclure que le complexe alpha37-57/antialpha se lie préférentiellement et de façon accrue, aux lymphocytes B qui sont des CPAgs porteuses de récepteur Fc.

### 7.2. Etude de la liaison aux splénocytes à l'aide du système protéique 14-4-4S/ZZDTR-BD décrit dans l'exemple 5.

Pour déterminer la population de splénocytes liée par le complexe 14-4-4S/ZZDTR-BD, une quantité fixe de ce complexe (10 nM) a été incubée en présence ou en absence de splénocytes et des quatre anticorps respectivement spécifiques des lymphocytes T CD4+, des lymphocytes T CD8+, des lymphocytes B et des molécules de CMH de classe II I-Ad et I-Ed. Ces anticorps sont marqués à la fluorescéine. Après 30 minutes à 4°C, les cellules ont été lavées et incubées en présence d'un anticorps polyclonal anti-anticorps de lapin couplé à la phycoerhytrine. 30 minutes plus tard, les cellules ont été lavées et analysées par cytométrie de flux.

Comme on peut le voir sur la figure 14, ZZDTR-BD interagit faiblement avec les lymphocytes TCD8+ (environ 12,4% des TCD8 sont marqués) et c'est aussi le cas du complexe 14-4-4/ZZDTR-BD (environ 13,3% des TCD8 sont marqués). ZZDTR-BD interagit faiblement avec les lymphocytes TCD4+ (environ 11,7% des TCD8 sont marqués) et c'est aussi le cas du complexe 14-4-4/ZZDTR-BD (environ 11% des TCD8 sont marqués). ZZDTR-BD interagit plus fortement avec les lymphocytes B (environ 50% des lymphocytes B sont marqués) et c'est aussi le cas du complexe 14-4-4/ZZDTR-BD (environ 68,5% des TCD8 sont marqués). L'anticorps de ciblage permet donc une augmentation de 37% du nombre de cellules B liées par ZZDTR-BD. Or les cellules B sont des CPAgs porteuses des molécules du CMH de classe II. Donc le complexe moléculaire 14-4-4S/ZZDTR-BD se lie préférentiellement aux CPAgs exprimant des molécules du CMH de classe II.

### Exemple 8 : Un composé possédant la capacité à cibler les HS et une molécule exprimée spécifiquement à la surface des CPAgs (14-4-4S/ZZDTR-BD) est capable d'induire une réponse immunitaire supérieure à celle induite par un composé qui possède la capacité à cibler une plus large variété de cellules (Mα2-3/ZZDTR-BD).

Pour évaluer si le ciblage des HS et d'un récepteur exprimé spécifiquement à la surface des CPAgs est capable d'induire une réponse immunitaire accrue « *in vivo* », l'immunogénicité de ZZDTR-BD lorsqu'il est complexé à l'anticorps 14-4-4S, qui cible les CPAgs exprimant les molécules de classe II, et lorsqu'il est complexé à un anticorps contrôle de même isotype (IgG2a), a été comparée.

Avant l'injection, la protéine de fusion ZZDTR-BD et les deux Acs ont été dilués en milieu HBSS. ZZDTR-BD a ensuite été incubée pendant une heure à température ambiante en présence de quantités équimolaires de l'anti-CMH ou de l'IgG2a contrôle. Deux groupes de quatre souris ont ensuite été injectés en absence d'adjuvant (0,01 nmole de complexe/souris/100µl) avec le complexe antiCMH/ZZDTR-BD ou avec le complexe IgG2a/ZZDTR-BD, respectivement. Quarante cinq jours après l'injection, le sang des animaux a été prélevé et les sérums ont été poolés. La présence d'anticorps anti-toxine diphtérique a ensuite été évaluée par dosage immunoenzymatique à l'aide de plaques de microtitration qui ont préalablement été adsorbées avec un mutant non-toxique de la toxine diphtérique, appelé CRM197, décrite dans Uchida et al., Science, 1972, 175, 901-903 (0,1 µg de CRM197/puits/100µl PBS). Pour réaliser cette évaluation, les deux sérums poolés ont été respectivement dilués et incubés une nuit à 4°C dans les plaques de microtitration. Les plaques ont ensuite été lavées puis incubées en présence d'un anticorps de chèvre anti-IgG de souris couplé à la péroxydase. Après 30 minutes, les plaques ont été lavées, un substrat (ABTS) a été ajouté, et la coloration a été mesurée à 414 nm après 30 minutes d'incubation supplémentaire. A partir de ces mesures, les titres en anticorps sont définis comme la dilution de sérum conduisant à une DO de 0,6.

Comme on peut le voir sur la figure 15, un titre de 1/3400 est mesuré pour le sérum issu de l'immunisation avec le complexe IgG2a/ZZDTR-BD ce qui indique que ce complexe est capable d'induire une réponse en anticorps anti-toxine diphtérique en absence d'adjuvant. Ce titre est cependant inférieur à celui mesuré pour le sérum issu de l'immunisation avec le complexe antiCMH/ZZDTR-BD (1/12000) ce qui indique que le ciblage des molécules de CMH de classe II permet d'accroître la réponse immunitaire humorale induite contre le complexe.

### Exemple 9: Un composé possédant la capacité à cibler les HS, une molécule exprimée spécifiquement à la surface des CPAgs et un épitope TCD8+ est capable d'induire une réponse immunitaire supérieure à un composé qui ne cible que les HS.

Pour évaluer si le double ciblage permet aussi d'augmenter la capacité à stimuler des cellules TCD8+ cytotoxiques plusieurs protéines de fusion ont été construites. La première protéine de fusion contient le double domaine ZZ de liaison aux immunoglobulines (SEQ ID NO: 3), un épitope TCD8+ de séquence SIINFEKL (SEQ ID NO : 10) qui est issu de l'ovalbumine (les séquences flanquantes LEQLE (SEQ ID NO: 11) et TEWTS (SEQ ID NO: 12) sont respectivement insérées en N- et C-terminal de cet épitope), un épitope TCD4+ de séquence SYKKVWRDHRGTI (SEQ ID NO: 13), le fragment Tat22-57_{C(22-37)S} qui contient la région de Tat capable de se lier aux héparanes sulfates. La deuxième protéine de fusion contient le double domaine ZZ de liaison aux immunoglobulines (SEQ ID NO: 3), un épitope TCD8+ de séquence SIINFEKL (SEQ ID NO : 10) qui est issu de l'ovalbumine (les séquences flanquantes LEQLE(SEQ ID NO: 11) et TEWTS (SEQ ID NO: 12) sont respectivement insérées en N- et C-terminal de cet épitope), un épitope TCD4+ de séquence SYKKVWRDHRGTI (SEQ ID NO: 13). La troisième protéine de fusion contient le double domaine ZZ de liaison aux immunoglobulines (SEQ ID NO: 3), l'ovalbumine, le fragment Tat22-57_{C(22-37)S} qui contient la région de Tat capable de se lier aux héparanes sulfates. La quatrième protéine de fusion contient le double domaine ZZ de liaison aux immunoglobulines et l'ovalbumine. L'ovalbumine est utilisée en contrôle. Ces protéines de fusion ont été utilisées libres ou complexées soit à l'anticorps monoclonal AF6-120.1 spécifique de la molécule de CMH de classe II I-Ab (# 553549; BECTON-DICKINSON BIOSCIENCES), soit à un anticorps monoclonal non spécifique mais de même sous-classe (IgG2a) que AF6-120.1, soit à un anticorps polyclonal de lapin antiIgG de souris, soit à un anticorps de lapin non spécifique. La formation des complexes a été réalisée dans des conditions identiques à celles décrites dans le sixième exemple.

Pour évaluer la capacité stimulante des différents composés, l'hybridome B3Z qui reconnaît l'épitope T de séquence SIINFEKL en association avec les molécules de classe I de type I-A^{b}, a été utilisé. Alternativement, des splénocytes issus de souris OTI qui contiennent des cellules T CD8+ qui reconnaissent aussi l'épitope SIINFEKL, ont été utilisés. Pour l'évaluation de la stimulation de l'hybridome B3Z, les protéines de fusion (antigènes) ont été incubées en présence ou en absence de chacun des quatre anticorps décrits précédemment. Les mélanges ont été ajoutés à des CPAgs. Les CPAgs sont, d'une part, une lignée de cellules dendritiques (5 10⁴/50µl/puits), d'autre part, des splénocytes (5 10⁵/50µl/puits) de souris C57B1/6.

Pour l'évaluation de la stimulation de l'hybridome B3Z, les antigènes (Ags) ont été incubés 2 h à 37 °C en présence de CPAgs, les cellules B3Z (5 10⁴/50µl/puits) ont été ajoutées et les mélanges incubés 24 h à 37 °C. La stimulation de la cellule B3Z est ensuite évaluée soit par mesure de la sécrétion d'interleukine 2, soit par mesure de l'expression du gène Lac Z qui code pour la β-galactosidase. La mesure de sécrétion d'IL-2 est réalisée à partir des surnageants de culture qui sont ensuite incubés sur une lignée CTL dont la croissance dépend de la présence d'IL-2. La mesure de l'activité β-galactosidase est réalisée en utilisant le chlorophenolred-β-D-galactopyranoside comme substrat.

Pour l'évaluation de la stimulation des cellules OTI, les Ags ont été incubés 5 h à 37 °C en présence des cellules dendritiques JAWSII. Les cellules ont ensuite été fixées à l'aide de glutaraldéhyde puis des splénocytes de souris OTI (5 10⁵/50µl/puits) ont été ajoutés et les mélanges incubés pendant 3 jours à 37 °C. De la thymidine tritiée a ensuite été ajoutée (1µCi/pts). Après 18 heures d'incubation à 37°C, les cellules ont été prélevées et la radioactivité incorporée aux cellules a été mesurée afin d'évaluer la prolifération des cellules OTI.

### Exemple 10: Un composé possédant la capacité à cibler les HS, une molécule exprimée spécifiquement à la surface des CPAgs et un Ag est capable d'induire une réponse immunitaire supérieure « in vivo » à un composé qui contient le même Ag mais ne cible que la molécule exprimée spécifiquement à la surface des CPAgs.

Pour évaluer si le double ciblage permet aussi d'augmenter la réponse immunitaire « *in vivo* » plusieurs protéines de fusion ont été construites. La première protéine de fusion contient le double domaine ZZ de liaison aux immunoglobulines (SEQ ID NO: 3), un épitope TCD8+ de séquence SIINFEKL (SEQ ID NO : 10) qui est issu de l'ovalbumine (les séquences flanquantes LEQLE (SEQ ID NO: 11) et TEWTS (SEQ ID NO: 12) sont respectivement insérées en N- et C-terminal de cet épitope), un épitope TCD4+ de séquence SYKKVWRDHRGTI (SEQ ID NO: 13), le fragment Tat22-57_{C(22-37)S} qui contient la région de Tat capable de se lier aux héparanes sulfates. La deuxième protéine de fusion contient le double domaine ZZ (SEQ ID NO: 3) de liaison aux immunoglobulines, un épitope TCD8+ de séquence SIINFEKL (SEQ ID NO : 10) qui est issu de l'ovalbumine (les séquences flanquantes LEQLE (SEQ ID NO: 11) et TEWTS (SEQ ID NO: 12) sont respectivement insérées en N- et C-terminal de cet épitope), un épitope TCD4+ de séquence SYKKVWRDHRGTI (SEQ ID NO: 13). La troisième protéine de fusion contient le double domaine ZZ de liaison aux immunoglobulines (SEQ ID NO: 3), l'ovalbumine, le fragment Tat22-57_{C(22-37)S} qui contient la région de Tat capable de se lier aux héparanes sulfates. La quatrième protéine de fusion contient le double domaine ZZ de liaison aux immunoglobulines (SEQ ID NO: 3) et l'ovalbumine. L'ovalbumine est utilisée en contrôle. Ces protéines de fusion ont été utilisées libres ou complexées soit à l'anticorps monoclonal AF6-120.1 spécifique de la molécule de CMH de classe II I-Ab (# 553549; BECTON-DICKINSON BIOSCIENCES), soit à un anticorps monoclonal non spécifique mais de même sous-classe (IgG2a) que AF6-120.1, soit à un anticorps polyclonal de lapin antiIgG de souris, soit à un anticorps de lapin non spécifique. La formation des complexes a été réalisée dans des conditions identiques à celles décrites dans le sixième exemple.

Pour évaluer la réponse immunitaire chez l'animal, les différents mélanges ont été injectés, en présence ou en absence d'adjuvant, chez la souris (6 animaux par groupe). Quatorze jours après la deuxième immunisation, le sang des animaux a été prélevé pour évaluer la réponse humorale. Les animaux ont ensuite été sacrifiés et leur rate a été prélevée pour évaluer la réponse cellulaire. La réponse humorale a été évaluée par dosage immunoenzymatique des anticorps anti-ovalbumine. Pour évaluer la réponse cellulaire, la présence de lymphocytes T spécifiques a été déterminée par comptage des cellules sécrétrices d'interféron gamma ou d'IL-4 à l'aide d'un test ELISPOT selon le protocole décrit dans Turbant et al., Vaccine, 2009, 27, 5349-56.

### Exemple 11: Un composé possédant la capacité à cibler les HS et une molécule exprimée spécifiquement à la surface des CPAgs est capable d'induire une réponse immunitaire cytotoxique supérieure à un composé qui ne cible que les HS ou que la molécule exprimée spécifiquement à la surface des CPAgs.

Pour évaluer si le double ciblage permet d'augmenter la capacité à stimuler des cellules TCD8+ cytotoxiques deux protéines chimériques ont été synthétisées chimiquement. La première protéine, appelée Tat47-57-SIINFEKL-alpha, contient la séquence de la toxine alpha de Naja nigricollis (SEQ ID NO: 9), un épitope TCD8+ de séquence SIINFEKL (SEQ ID NO : 10) qui est issu de l'ovalbumine (les séquences flanquantes LEQLE (SEQ ID NO: 11) et TEWTS (SEQ ID NO: 12) sont respectivement insérées en N- et C-terminal de cet épitope), le fragment Tat47-57 (SEQ ID NO :14) qui contient la région de Tat capable de se lier aux héparanes sulfates. La seconde protéine, appelée SIINFEKL-alpha, contient la séquence de la toxine alpha de Naja nigricollis (SEQ ID NO: 9), un épitope TCD8+ de séquence SIINFEKL (SEQ ID NO : 10) qui est issu de l'ovalbumine (les séquences flanquantes LEQLE (SEQ ID NO: 11) et TEWTS (SEQ ID NO: 12) sont respectivement insérées en N- et C-terminal de cet épitope). Ces deux protéines ont été utilisées libres ou complexées à l'anticorps polyclonal de lapin anti-toxine alpha, appelé anti-alpha, décrit dans l'exemple 3. La formation des complexes a été réalisée par incubation une nuit à 4°C.

Pour évaluer la capacité stimulante des deux composés, des splénocytes de souris OTI ont été utilisés. Ces splénocytes contiennent des lymphocytes TCD8+ qui reconnaissent l'épitope T, de séquence SIINFEKL, en association avec les molécules de classe I de type I-A^{b}. Les deux protéines (dilution 1µM final) complexées ou non complexées à l'anticorps (50nM) ont été respectivement incubées dans des plaques de culture cellulaire en présence d'une lignée de cellules dendritiques utilisée comme CPAg. Cette lignée, appelée JAWSII, a été incubée à raison de 3 10⁴/50µl/puits. Après 5 h à 37 °C, les plaques ont été lavées et les cellules ont été fixées à l'aide de glutaraldéhyde. Après fixation, des splénocytes de souris OTI ont été ajoutés (9 10⁴/50µl/puits). Après 4 jours d'incubation à 37 °C, une solution de thymidine tritiée a été ajoutée (1µCi/25µl/puits) et les plaques ont été incubées pendant 18 heures à 37°C. Les cellules ont ensuite été prélevées et la radioactivité a été mesurée pour évaluer la prolifération cellulaire. Comme on peut le voir sur la figure 16, le composé SIINFEKL-alpha libre est capable d'induire la prolifération des cellules T-CD8+. En présence d'une concentration fixe d'anti-alpha (50nM final), SIINFEKL-alpha stimule plus fortement les cellules OTI ce qui indique que le ciblage médié par les récepteurs Fcy permet aussi d'augmenter la présentation croisée, comme cela avait été observé précédemment pour d'autres antigènes (Regnault, J. Exp. Med., 1999, 371-380). Un accroissement de la stimulation des cellules OTI est aussi observé pour le composé Tat47-57-SIINFEKL-alpha ce qui indique que le ciblage médié par liaison aux HS permet d'accroitre la présentation croisée. L'accroissement le plus important de la stimulation des cellules OTI est cependant observé lorsque Tat47-57-SIINFEKL-alpha est complexé avec l'anticorps anti-alpha ce qui indique que le ciblage médié conjointement par les HS et les récepteurs Fc procure un effet synergique sur la présentation croisée de la protéine antigénique.

### Exemple 12: Un composé possédant la capacité à cibler les HS et une molécule exprimée spécifiquement à la surface des CPAgs accroît plus fortement l'expression des molécules de co-stimulation CD80 et CD86 que les composés qui ne ciblent que les HS ou que la molécule exprimée spécifiquement à la surface des CPAgs.

La capacité de présentation des antigènes représente un aspect crucial dans la mise en place d'une réponse immunitaire adaptative. Cependant, la mise en place de cette réponse requiert aussi l'activation des cellules présentatrices de l'antigène (CPAgs). C'est pourquoi la capacité des complexes moléculaires de ciblage d'activer des CPAgs a été évaluée. Pour évaluer cet aspect, laptitude du complexe moléculaire de ciblage décrit dans l'exemple 11 à augmenter l'expression des molécules de co-stimulation CD80 et CD86 par les CPAgs JAWSII, décrites précédemment, a été analysée. Les protéines chimériques et anticorps utilisés pour réaliser cette évaluation sont celles décrites dans l'exemple 11. Les deux protéines ont été utilisées libres ou complexées à l'anticorps polyclonal de lapin anti-toxine alpha. La formation des complexes a été réalisée par incubation une nuit à 4°C. La toxine alpha sauvage a aussi été utilisée, à titre de contrôle.

Les deux protéines (1µM final) complexées ou non complexées à l'anticorps (25nM final) ont été respectivement diluées et incubées dans des plaques de culture cellulaire en présence de la lignée JAWSII (5.10⁵/100µl/puits) pendant 24 heures à 37°C. Les cellules ont ensuite été lavées et incubées 30 minutes à 4°C en présence d'anticorps anti-CD80 marqué à la fluorescéine et anti-CD86 marqué à la fluorescéine, respectivement. La liaison des anticorps aux cellules a enfin été analysée par cytométrie de flux. Comme on peut le voir sur la figure 17A, lorsque les cellules ont été incubées en présence de SIINFEKL-alpha, la molécule CD80 est exprimée à la surface des CPAgs. L'expression de la molécule CD80 est accrue lorsque les JAWSII ont été incubées avec Tat47-57-SIINFEKL-alpha ou avec le complexe SIINFEKL-alpha/anti-alpha. L'expression de CD80 est cependant plus fortement accrue pour le complexe Tat47-57-SIINFEKL-alpha/anti-alpha ce qui indique que le complexe moléculaire de ciblage procure une synergie d'effet. Le même comportement est observé pour la molécule CD86 (cf figure 17B). Ainsi, lorsque les cellules ont été incubées en présence de SIINFEKL-alpha, la molécule CD86 est exprimée à la surface des CPAgs. L'expression de la molécule CD86 est accrue lorsque les JAWSII ont été incubées avec Tat47-57-SIINFEKL-alpha ou avec le complexe SIINFEKL-alpha/anti-alpha. L'expression de CD86 est cependant plus fortement accrue pour le complexe Tat47-57-SIINFEKL-alpha/antialpha ce qui indique que le complexe moléculaire de ciblage procure une synergie d'effet.

Cet exemple qui démontre l'effet synergique sur l'expression des molécules de co-stimulation CD80 et CD86, indique donc que le complexe moléculaire de ciblage provoque une activation des cellules présentatrices de l'Ag.

## Revendications

1. Complexe moléculaire, **caractérisé en ce qu'**il comprend au moins un antigène associé à au moins deux ligands de molécules de surface de cellules présentatrices d'antigène,
ledit complexe comprenant au moins un premier ligand d'un héparane sulfate sélectionné dans le groupe constitué par : les peptides et les protéines qui lient les héparanes sulfates, les anticorps naturels ou recombinant dirigés contre les héparanes sulfates et les fragments desdits anticorps contenant au moins le paratope, et
un deuxième ligand d'une molécule de surface spécifique des cellules présentatrices d'antigène sélectionné dans le groupe constitué par : les anticorps spécifiques dudit antigène et leurs fragments comprenant au moins la région Fc, et les protéines et les fragments de protéines qui lient la région Fc et/ou Fab des anticorps,
ledit antigène étant une protéine ou un peptide, et
ledit complexe comprenant une protéine de fusion ou un conjugué covalent du premier ligand avec l'antigène ou du premier ligand avec l'antigène et le deuxième ligand.

2. Complexe moléculaire, **caractérisé en ce qu'**il comprend au moins un antigène associé à au moins deux ligands de molécules de surface de cellules présentatrices d'antigène,
ledit complexe comprenant au moins un premier ligand d'un héparane sulfate sélectionné dans le groupe constitué par : les peptides et les protéines qui lient les héparanes sulfates, les anticorps naturels ou recombinant dirigés contre les héparanes sulfates et les fragments desdits anticorps contenant au moins le paratope, et
un deuxième ligand d'une molécule de surface spécifique des cellules présentatrices d'antigène sélectionné dans le groupe constitué par : les anticorps dirigés contre lesdites molécules de surface spécifiques des cellules présentatrices d'antigène et leurs fragments contenant au moins le paratope, et les anticorps non-spécifiques dudit antigène et leurs fragments comprenant au moins la région Fc,
ledit antigène étant une protéine ou un peptide, et
ledit complexe comprenant une protéine de fusion ou un conjugué covalent du premier ligand avec l'antigène et le deuxième ligand.

3. Complexe moléculaire, **caractérisé en ce qu'**il comprend au moins un antigène associé à au moins deux ligands de molécules de surface de cellules présentatrices d'antigène,
ledit complexe comprenant au moins un premier ligand d'un héparane sulfate sélectionné dans le groupe constitué par : les peptides et les protéines qui lient les héparanes sulfates, les anticorps naturels ou recombinant dirigés contre les héparanes sulfates et les fragments desdits anticorps contenant au moins le paratope, et
un deuxième ligand d'une molécule de surface spécifique des cellules présentatrices d'antigène sélectionné dans le groupe constitué par : les anticorps dirigés contre lesdites molécules de surface spécifiques des cellules présentatrices d'antigène et leurs fragments contenant au moins le paratope, et les anticorps non-spécifiques dudit antigène et leurs fragments comprenant au moins la région Fc,
ledit antigène étant une protéine ou un peptide, et
ledit complexe comprenant une protéine de fusion ou un conjugué covalent du premier ligand avec l'antigène et un élément de liaison constitué par une protéine ou un fragment de protéine qui lie la région Fc et/ou Fab des anticorps, et ledit élément de liaison étant lié de façon covalente à l'antigène, à la protéine de fusion ou au conjugué du premier ligand avec l'antigène.

4. Complexe moléculaire selon l'une quelconque des revendications 1 à 3, **caractérisé en ce que** ledit antigène est un antigène spécifique d'un agent pathogène ou d'une tumeur.

5. Complexe moléculaire selon l'une quelconque des revendications 1 à 4, **caractérisé en ce que** la molécule de surface spécifique des cellules présentatrices d'antigène est sélectionnée dans le groupe constitué par : les molécules du CMH de classe II, les récepteurs des lectines de type C, les immunoglobulines membranaires, et les récepteurs pour la région constante des immunoglobulines.

6. Complexe moléculaire selon l'une quelconque des revendications 1 à 5, **caractérisé en ce que** le premier ligand est un peptide sélectionné dans le groupe constitué par : un fragment de la protéine Tat du VIH, comprenant au moins la région basique Tat 49-57, tel que les peptides Tat 49-57 (SEQ ID NO: 1) et Tat 37-57 (SEQ ID NO : 7), un peptide polyarginine R7 à R11, et un peptide comprenant le domaine R de la toxine diphtérique (SEQ ID NO : 6).

7. Complexe moléculaire selon l'une quelconque des revendications 1 à 6, **caractérisé en ce que** lesdits fragments d'anticorps contenant au moins le paratope sont sélectionnés dans le groupe constitué par les fragments Fab, Fab', F(ab')₂, Fv, scFv, Fabc et Fab avec au moins une portion du domaine Fc.

8. Complexe moléculaire selon l'une quelconque des revendications 1 et 3 à 7, **caractérisé en ce que** ladite protéine ou fragment de protéine qui lie la région Fc et/ou Fab des anticorps est sélectionnée dans le groupe constitué par la protéine A de *Staphylococcus aureus,* son fragment BB (SEQ ID NO: 4) et son dérivé ZZ (SEQ ID NO: 3).

9. Complexe moléculaire selon l'une quelconque des revendications 6 à 8, **caractérisé en ce qu'**il comprend : (i) un antigène lié de façon covalente à un peptide tel que défini à la revendication 6 (premier ligand), (ii) un anticorps dirigé contre ledit antigène (deuxième ligand), et éventuellement une protéine ou un fragment de protéine qui lie la région Fab des anticorps telle que la protéine A de *Staphylococcus aureus.*

10. Complexe moléculaire selon l'une quelconque des revendications 6 à 8, **caractérisé en ce qu'**il comprend : (i) un antigène lié de façon covalente à un peptide tel que défini à la revendication 6 (premier ligand), et à une protéine ou un fragment de protéine qui lie la région Fc et/ou Fab des anticorps, de préférence uniquement la région Fab, tels que la protéine A de *Staphylococcus aureus* et son fragment BB (deuxième ligand ou élément de liaison), et lorsque ladite protéine ou ledit fragment de protéine qui lie la région Fc et/ou Fab des anticorps, est un élément de liaison, ledit complexe comprend également (ii) un anticorps, de préférence une IgG, ou un fragment dudit anticorps comprenant au moins la région Fc (deuxième ligand).

11. Complexe moléculaire selon la revendication 6 ou 7, **caractérisé en ce qu'**il comprend un antigène lié de façon covalente à un peptide tel que défini à la revendication 6 (premier ligand), et à un anticorps sélectionné dans le groupe constitué par un anticorps anti-CMH de classe II, un anticorps anti-RFcgamma et un anticorps anti-DEC-205, ou bien à un fragment des anticorps précédents comprenant au moins le paratope (deuxième ligand).

12. Complexe moléculaire selon l'une quelconque des revendications 6 à 8, **caractérisé en ce qu'**il comprend : (i) un antigène lié de façon covalente à un peptide tel que défini à la revendication 6 (premier ligand) et à une protéine ou un fragment de protéine qui lie la région Fc et/ou Fab des anticorps tels que le fragment BB de la protéine A de *Staphylococcus aureus* et son dérivé ZZ (élément de liaison), et (ii) un anticorps sélectionné dans le groupe constitué par un anticorps anti-CMH de classe II, un anticorps anti-RFcgamma, et un anticorps anti-DEC-205, ou un fragment des anticorps précédents comprenant au moins le paratope (deuxième ligand).

13. Vecteur recombinant isolé ou mélange de vecteurs recombinants, comprenant au moins les séquences codant pour l'antigène, le premier et le second ligand tels que définis à l'une quelconque des revendications 1 à 4, 6, et 9 à 12, insérées dans un polynucléotide ou une construction polynucléotidique isolés ou un mélange de polynucléotides ou de constructions polynucléotidiques, sélectionnés dans le groupe constitué par :
a) un polynucléotide isolé comprenant une séquence codant pour une protéine de fusion comprenant au moins les séquences codantes de l'antigène, du premier et du second ligands tels que définis à l'une quelconque des revendications 1 à 4, 6, et 9 à 12, fusionnées en phase de façon appropriée,
b) un mélange de polynucléotides comprenant au moins un premier polynucléotide comprenant une séquence codant pour une protéine de fusion comprenant au moins les séquences codantes de l'antigène et du premier ligand tels que définis à l'une quelconque des revendications 1 à 4, 6 et 9 à 12, fusionnées en phase de façon appropriée et un second polynucléotide comprenant une séquence codant pour le deuxième ligand tel que défini à l'une quelconque des revendications 1 à 3, et 9 à 12, ledit premier ou second polynucléotide comprenant également une séquence codant pour un élément de liaison tel que défini à la revendication 3,
c) une construction polynucléotide isolée ou un mélange de constructions polynucléotidiques comprenant au moins le polynucléotide tel que défini en a) ou les deux polynucléotides tels que définis en b), liés de façon opérationnelle à des séquences régulatrices de la transcription et/ou de la traduction appropriées pour l'expression du premier ligand, de l'antigène et/ou du second ligand dans des cellules hôtes.

14. Vecteur recombinant ou mélange de vecteurs recombinants selon la revendication 13, **caractérisé en ce qu'**il s'agit d'un plasmide d'expression ou d'un mélange de plasmides d'expression.

15. Complexe moléculaire selon l'une quelconque des revendications 1 à 12, comme vaccin pour la prévention ou le traitement d'une maladie infectieuse ou d'un cancer.

## Patentansprüche

1. Molekülkomplex, **dadurch gekennzeichnet, dass** er mindestens ein Antigen umfasst, assoziiert mit mindestens zwei Liganden von Oberflächenmolekülen von antigenpräsentierenden Zellen,
wobei der Komplex mindestens einen ersten Liganden eines Heparansulfats umfasst, ausgewählt aus der Gruppe bestehend aus: den Peptiden und den Proteinen, die die Heparansulfate binden, den natürlichen oder rekombinanten Antikörpern, die gegen die Heparansulfate gerichtet sind, und die Fragmente dieser Antikörper, umfassend mindestens das Paratop, und
einen zweiten Liganden eines Oberflächenmoleküls, das für die antigenpräsentierenden Zellen spezifisch ist, ausgewählt aus der Gruppe bestehend aus: den Antikörpern die für dieses Antigen spezifisch sind und ihre Fragmente, umfassend mindestens die Fc Region, und die Proteine und die Fragmente der Proteine, die die Fc und/oder Fab Region des Antikörpers binden,
wobei das Antigen ein Protein oder ein Peptid ist, und
wobei der Komplex ein Fusionsprotein oder ein kovalentes Konjugat des ersten Liganden mit dem Antigen oder des ersten Liganden mit dem Antigen und dem zweiten Liganden umfasst.

2. Molekülkomplex, **dadurch gekennzeichnet, dass** er mindestens ein Antigen umfasst, assoziiert mit mindestens zwei Liganden von Oberflächenmolekülen von antigenpräsentierenden Zellen,
wobei der Komplex mindestens einen ersten Liganden eines Heparansulfats umfasst, ausgewählt aus der Gruppe bestehend aus: den Peptiden und den Proteinen, die die Heparansulfate binden, den natürlichen oder rekombinanten Antikörpern, die gegen die Heparansulfate gerichtet sind, und die Fragmente dieser Antikörper, umfassend mindestens das Paratop, und
einen zweiten Liganden eines Oberflächenmoleküls, das für die antigenpräsentierenden Zellen spezifisch ist, ausgewählt aus der Gruppe bestehend aus: den Antikörpern die gegen die Oberflächenmoleküle, die für die antigenpräsentierenden Zellen spezifisch sind, gerichtet sind und ihre Fragmente, umfassend mindestens das Paratop, und die Antikörper, die für das Antigen nicht spezifisch sind und ihre Fragmente, umfassend mindestens die Fc Region,
wobei das Antigen ein Protein oder ein Peptid ist, und
wobei der Komplex ein Fusionsprotein oder ein kovalentes Konjugat des ersten Liganden mit dem Antigen und dem zweiten Liganden umfasst.

3. Molekülkomplex, **dadurch gekennzeichnet, dass** er mindestens ein Antigen umfasst, assoziiert mit mindestens zwei Liganden von Oberflächenmolekülen von antigenpräsentierenden Zellen,
wobei der Komplex mindestens einen ersten Liganden eines Heparansulfats umfasst, ausgewählt aus der Gruppe bestehend aus: den Peptiden und den Proteinen, die die Heparansulfate binden, den natürlichen oder rekombinanten Antikörpern, die gegen die Heparansulfate gerichtet sind, und die Fragmente dieser Antikörper, umfassend mindestens das Paratop, und
einen zweiten Liganden eines Oberflächenmoleküls, das für die antigenpräsentierenden Zellen spezifisch ist, ausgewählt aus der Gruppe bestehend aus: den Antikörpern, die gegen die Oberflächenmoleküle, die für die antigenpräsentierenden Zellen spezifisch sind, gerichtet sind und ihre Fragmente, umfassend mindestens das Paratop, und die Antikörper, die für das Antigen nicht spezifisch sind, und ihre Fragmente, umfassend mindestens die Fc Region,
wobei das Antigen ein Protein oder ein Peptid ist, und
wobei der Komplex ein Fusionsprotein oder ein kovalentes Konjugat des ersten Liganden mit dem Antigen und einem Bindungselement umfasst, gebildet aus einem Protein oder einem Proteinfragment, das die Fc und/oder Fab Region der Antikörper bindet, und wobei das Bindungselement in einer kovalenten Weise an das Antigen, das Fusionsprotein oder das Konjugat des ersten Liganden mit dem Antigen gebunden ist.

4. Molekülkomplex nach einem der Ansprüche 1 bis 3, **dadurch gekennzeichnet, dass** das Antigen ein für ein Pathogen oder einen Tumor spezifisches Antigen ist.

5. Molekülkomplex nach einem der Ansprüche 1 bis 4, **dadurch gekennzeichnet, dass** das Oberflächenmolekül, das für die antigenpräsentierenden Zellen spezifisch ist, ausgewählt ist aus der Gruppe bestehend aus: den Klasse II MHC-Molekülen, den Rezeptoren der Typ-C Lectine, den Membran-Immunglobulinen und den Rezeptoren für die konstante Region der Immunglobuline.

6. Molekülkomplex nach einem der Ansprüche 1 bis 5, **dadurch gekennzeichnet, dass** der erste Ligand ein Peptid ist, ausgewählt aus der Gruppe bestehend aus: einem Fragment des Tat-Proteins von HIV, umfassend mindestens die basische Region Tat 49-57, wie die Peptide Tat 49-57 (SEQ ID NO: 1) und Tat 37-57 (SEQ ID NO: 7), ein Polyargininpeptid R7 bis R11 und ein Peptid, umfassend die R-Domäne des Diphtherietoxins (SEQ ID NO: 6).

7. Molekülkomplex nach einem der Ansprüche 1 bis 6, **dadurch gekennzeichnet, dass** die Fragmente der Antikörper, umfassend mindestens das Paratop, ausgewählt sind aus der Gruppe bestehend aus den Fragmenten Fab, Fab', F(ab')₂, Fv, scFv, Fabc und Fab mit mindestens einem Teil der Fc Domäne.

8. Molekülkomplex nach einem der Ansprüche 1 und 3 bis 7, **dadurch gekennzeichnet, dass** das Protein oder Proteinfragment, das die Fc und/oder Fab Region der Antikörper bindet, ausgewählt ist aus der Gruppe, bestehend aus dem Protein A von *Staphylococcus aureus,* seinem BB Fragment (SEQ ID NO: 4) und seinem ZZ Derivat (SEQ ID NO: 3).

9. Molekülkomplex nach einem der Ansprüche 6 bis 8, **dadurch gekennzeichnet, dass** er umfasst: (i) ein Antigen, das in einer kovalenten Weise an ein Peptid wie in Anspruch 6 definiert gebunden ist (erster Ligand), (ii) einen Antikörper, der gegen das Antigen gerichtet ist (zweiter Ligand) und gegebenenfalls ein Protein oder ein Proteinfragment, das die Fab Region der Antikörper bindet, wie das Protein A von *Staphylococcus aureus.*

10. Molekülkomplex nach einem der Ansprüche 6 bis 8, **dadurch gekennzeichnet, dass** er umfasst: (i) ein Antigen, das in einer kovalenten Weise an ein Peptid, wie in Anspruch 6 definiert (erster Ligand), und an ein Protein oder ein Proteinfragment gebunden ist, das die Fc und/oder Fab Region des Antikörpers, vorzugsweise nur die Fab Region, bindet, wie das Protein A von *Staphylococcus aureus* und sein BB Fragment (zweiter Ligand oder Bindungselement), und wenn das Protein oder das Proteinfragment, das die Fc und/oder Fab Region der Antikörper bindet, ein Bindungselement ist, umfasst der Komplex auch (ii) einen Antikörper, vorzugsweise ein IgG, oder ein Fragment des Antikörpers, umfassend mindestens die Fc Region (zweiter Ligand).

11. Molekülkomplex nach Anspruch 6 oder 7, **dadurch gekennzeichnet, dass** er ein Antigen umfasst, das in einer kovalenten Weise an ein Peptid, wie in Anspruch 6 definiert (erster Ligand), und an einen Antikörper, ausgewählt aus der Gruppe bestehend aus einem Klasse II anti-MHC-Antikörper, einem anti-RFcgamma-Antikörper und einem anti-DEC-205-Antikörper, oder an ein Fragment der obigen Antikörper, umfassend mindestens das Paratop (zweiter Ligand), gebunden ist.

12. Molekülkomplex nach einem der Ansprüche 6 bis 8, **dadurch gekennzeichnet, dass** er umfasst: (i) ein Antigen, das in einer kovalenten Weise an ein Peptid, wie in Anspruch 6 definiert (erster Ligand), und an ein Protein oder ein Proteinfragment, das die Fc und/oder Fab Region der Antikörper bindet, wie das BB Fragment des Proteins A von *Staphylococcus aureus* und sein ZZ Derivat (Bindungselement), gebunden ist und (ii) einen Antikörper, ausgewählt aus der Gruppe bestehend aus einem Klasse II anti-MHC-Antikörper, einem anti-RFcgamma-Antikörper und einem anti-DEC-205-Antikörper oder einem Fragment der obigen Antikörper, umfassend mindestens das Paratop (zweiter Ligand).

13. Isolierter rekombinanter Vektor oder Mischung rekombinanter Vektoren, umfassend mindestens die Sequenzen, die für das Antigen, den ersten und den zweite Liganden, wie in einem der Ansprüche 1 bis 4, 6, und 9 bis 12 definiert, kodieren, eingefügt in ein isoliertes Polynukleotid oder Polynukleotidkonstrukt oder eine Mischung aus Polynukleotiden oder Polynukleotidkonstrukten, ausgewählt aus der Gruppe, bestehend aus:
a) einem isolierten Polynukleotid, das eine Sequenz umfasst, die für ein Fusionsprotein kodiert, umfassend mindestens die kodierenden Sequenzen des Antigens, des ersten und des zweiten Liganden, wie in einem der Ansprüche 1 bis 4, 6, und 9 bis 12 definiert, in geeigneter Weise in Phase fusioniert,
b) eine Mischung von Polynukleotiden, umfassend mindestens ein erstes Polynukleotid, umfassend eine Sequenz, die für ein Fusionsprotein kodiert, umfassend mindestens die kodierenden Sequenzen des Antigens und des ersten Liganden, wie in einem der Ansprüche 1 bis 4, 6 und 9 bis 12 definiert, in geeigneter Weise in Phase fusioniert, und ein zweites Polynukleotid, umfassend eine Sequenz, die für den zweiten Liganden, wie in einem der Ansprüche 1 bis 3 und 9 bis 12 definiert, kodiert, wobei das erste oder zweite Polynukleotid auch eine Sequenz umfasst, die für ein Bindungselement, wie in Anspruch 3 definiert, kodiert,
c) ein isoliertes Polynukleotidkonstrukt oder eine Mischung von Polynukleotidkonstrukten, umfassend mindestens ein Polynukleotid, wie in a) definiert, oder die zwei Polynukleotide, wie in b) definiert, in operativer Weise gebunden an die Transkriptionsregulierungssequenzen und/oder Translationsregulierungssequenzen, die für die Expression des ersten Liganden, des Antigens und/oder des zweiten Liganden in der Wirtszelle geeignet sind.

14. Rekombinanter Vektor oder Mischung rekombinanter Vektoren nach Anspruch 13, **dadurch gekennzeichnet, dass** es sich um ein Expressionsplasmid oder eine Mischung von Expressionsplasmiden handelt.

15. Molekülkomplex nach einem der Ansprüche 1 bis 12, als Impfstoff zur Vorbeugung oder Behandlung einer Infektionskrankheit oder eines Krebses.

## Claims

1. Molecular complex, **characterised in that** it comprises at least one antigen associated with at least two ligands of surface molecules of antigen-presenting cells, said complex comprising at least one first ligand of a heparan sulphate selected from the group consisting of: the peptides and proteins which bind heparan sulphates, the natural or recombinant antibodies directed against heparan sulphates and the fragments of said antibodies containing at least the paratope, and
a second ligand of a surface molecule specific for antigen-presenting cells selected from the group consisting of: the antibodies specific for said antigen and their fragments comprising at least the Fc region, and the proteins and protein fragments which bind the antibody Fc and/or Fab region,
said antigen being a protein or a peptide, and
said complex comprising a fusion protein or a covalent conjugate of the first ligand with the antigen or of the first ligand with the antigen and the second ligand.

2. Molecular complex, **characterised in that** it comprises at least one antigen associated with at least two ligands of surface molecules of antigen-presenting cells, said complex comprising at least one first ligand of a heparan sulphate selected from the group consisting of: the peptides and proteins which bind heparan sulphates, the natural or recombinant antibodies directed against heparan sulphates and the fragments of said antibodies containing at least the paratope, and
a second ligand of a surface molecule specific for antigen-presenting cells selected from the group consisting of: the antibodies directed against said surface molecules specific for antigen-presenting cells and their fragments containing at least the paratope, and the antibodies not specific for said antigen and their fragments comprising at least the Fc region,
said antigen being a protein or a peptide, and
said complex comprising a fusion protein or a covalent conjugate of the first ligand with the antigen and the second ligand.

3. Molecular complex, **characterised in that** it comprises at least one antigen associated with at least two ligands of surface molecules of antigen-presenting cells, said complex comprising at least one first ligand of a heparan sulphate selected from the group consisting of: the peptides and proteins which bind heparan sulphates, the natural or recombinant antibodies directed against heparan sulphates and the fragments of said antibodies containing at least the paratope, and
a second ligand of a surface molecule specific for antigen-presenting cells selected from the group consisting of: the antibodies directed against said surface molecules specific for the antigen-presenting cells and their fragments containing at least the paratope, and the antibodies not specific for said antigen and their fragments comprising at least the Fc region,
said antigen being a protein or a peptide, and
said complex comprising a fusion protein or a covalent conjugate of the first ligand with the antigen and a binding element consisting of a protein or a protein fragment which binds the antibody Fc and/or Fab region, and said binding element being covalently bound to the antigen, to the fusion protein or to the conjugate of the first ligand with the antigen.

4. Molecular complex according to any one of claims 1 to 3, **characterised in that** said antigen is an antigen specific for a pathogenic agent or of a tumour.

5. Molecular complex according to any one of claims 1 to 4, **characterised in that** the surface molecule specific for antigen-presenting cells is selected from the group consisting of: MHC class II molecules, C-type lectin receptors, membrane immunoglobulins, and immunoglobulin constant region receptors.

6. Molecular complex according to any one of claims 1 to 5, **characterised in that** the first ligand is a peptide selected from the group consisting of: a fragment of the Tat protein of HIV comprising at least the Tat 49-57 basic region, such as the Tat 49-57 (SEQ ID NO: 1) and Tat 37-57 (SEQ ID NO: 7) peptides, a polyarginine peptide R7 to R11, and a peptide comprising the R domain of diphtheria toxin (SEQ ID NO: 6).

7. Molecular complex according to any one of claims 1 to 6, **characterised in that** said fragments of antibodies containing at least the paratope are selected from the group consisting of the Fab, Fab', F(ab')₂, Fv, scFv fragments, Fabc fragment and Fab fragment with at least a portion of the Fc domain.

8. Molecular complex according to any one of claims 1 and 3 to 7, **characterised in that** said protein or protein fragment which binds the antibody Fc and/or Fab region is selected from the group consisting of *Staphylococcus aureus* protein A, the fragment BB thereof (SEQ ID NO: 4) and the ZZ derivative thereof (SEQ ID NO: 3).

9. Molecular complex according to any one of claims 6 to 8, **characterised in that** it comprises: (i) an antigen covalently bound to a peptide as defined in claim 6 (first ligand), (ii) an antibody directed against said antigen (second ligand), and optionally a protein or a protein fragment which binds the antibody Fab region such as *Staphylococcus aureus* protein A.

10. Molecular complex according to any one of claims 6 to 8, **characterised in that** it comprises: (i) an antigen covalently bound to a peptide as defined in claim 6 (first ligand), and to a protein or a protein fragment which binds the antibody Fc and/or Fab region, preferably only the Fab region, such as *Staphylococcus aureus* protein A and the BB fragment thereof (second ligand or binding element), and when said protein or said protein fragment which binds the antibody Fc and/or Fab region is a binding element, said complex also comprises (ii) an antibody, preferably an IgG, or a fragment of said antibody comprising at least the Fc region (second ligand).

11. Molecular complex according to claim 6 or 7, **characterised in that** it comprises an antigen covalently bound to a peptide as defined in claim 6 (first ligand), and to an antibody selected from the group consisting of an anti-MHC class II antibody, an anti-FcgammaR antibody and an anti-DEC-205 antibody, or to a fragment of the preceding antibodies comprising at least the paratope (second ligand).

12. Molecular complex according to any one of claims 6 to 8, **characterised in that** it comprises: (i) an antigen covalently bound to a peptide as defined in claim 6 (first ligand) and to a protein or a protein fragment which binds the antibody Fc and/or Fab region such as the BB fragment of *Staphylococcus aureus* protein A of and the ZZ derivative thereof (binding element), and (ii) an antibody selected from the group consisting of an anti-MHC class II antibody, an anti-FcgammaR antibody, and an anti-DEC-205 antibody, or a fragment of the preceding antibodies comprising at least the paratope (second ligand).

13. An isolated recombinant vector or a mixture of recombinant vectors, comprising at least the sequences coding for the antigen, the first and the second ligand as defined in any one of claims 1 to 4, 6, and 9 to 12, inserted into an isolated polynucleotide or polynucleotide construct or a mixture of polynucleotides or of polynucleotide constructs, selected from the group consisting of:
a) an isolated polynucleotide comprising a sequence coding for a fusion protein comprising at least the coding sequences of the antigen, of the first and of the second ligand as defined in any one of claims 1 to 4, 6, and 9 to 12, fused in phase in a suitable manner,
b) a mixture of polynucleotides comprising at least a first polynucleotide comprising a sequence coding for a fusion protein comprising at least the coding sequences of the antigen and of the first ligand as defined in any one of claims 1 to 4, 6, and 9 to 12, fused in phase in a suitable manner and a second polynucleotide comprising a sequence coding for the second ligand as defined in any one of claims 1 to 3, and 9 to 12, said first or second polynucleotide also comprising a sequence coding for a binding element as defined in claim 3,
c) an isolated polynucleotide construct or a mixture of polynucleotide constructs comprising at least the polynucleotide as defined in a) or the two polynucleotides as defined in b), operationally linked to regulatory sequences for transcription and/or translation suitable for the expression of the first ligand, of the antigen and/or of the second ligand in host cells.

14. Recombinant vector or mixture of recombinant vectors according to claim 13, **characterised in that** it is an expression plasmid or a mixture of expression plasmids.

15. Molecular complex according to any one of claims 1 to 12, as a vaccine for the prevention or the treatment of an infectious disease or of a cancer.


### SEQUENCE LISTING

<110> COMMISSARIAT A L'ENERGIE ATOMIQUE ET AUX ENERGIES ALTERNATIVES LEONETTI Michel SAVATIER Alexandra GADZINSKI Adeline BOULAIN Jean-Claude
<120> COMPLEXE MOLECULAIRE DE CIBLAGE DES ANTIGENES VERS LES CELLULES PRESENTATRICES D'ANTIGENE ET SES APPLICATIONS POUR LA VACCINATION
<130> 263FR373
<160> 14
<170> PatentIn version 3.5
<210> 1
   <211> 9
   <212> PRT
   <213> artificial sequence
<220>
   <223> HIV-1 Tat 49-57 peptide (basic region)
<400> 1
   Arg Lys Lys Arg Arg Gln Arg Arg Arg
   1               5
<210> 2
   <211> 16
   <212> PRT
   <213> artificial sequence
<220>
   <223> penetratin peptide : Antennapedia protein homeodomain peptide 43-58
<400> 2
   Arg Gln Ile Lys Ile Trp Phe Gln Asn Arg Arg Met Lys Trp Lys Lys
   1               5                   10                  15
<210> 3
   <211> 116
   <212> PRT
   <213> artificial sequence
<220>
   <223> ZZ derivative of S.aureus Protein A BB domain
<400> 3
   Val Asp Asn Lys Phe Asn Lys Glu Gln Gln Asn Ala Phe Tyr Glu Ile
   1               5                   10                  15
   Leu His Leu Pro Asn Leu Asn Glu Glu Gln Arg Asn Ala Phe Ile Gln
               20                  25                  30
   Ser Leu Lys Asp Asp Pro Ser Gln Ser Ala Asn Leu Leu Ala Glu Ala
           35                  40                  45
   Lys Lys Leu Asn Asp Ala Gln Ala Pro Lys Val Asp Asn Lys Phe Asn
       50                  55                  60
   Lys Glu Gln Gln Asn Ala Phe Tyr Glu Ile Leu His Leu Pro Asn Leu
   65                  70                  75                  80
   Asn Glu Glu Gln Arg Asn Ala Phe Ile Gln Ser Leu Lys Asp Asp Pro
                   85                  90                  95
   Ser Gln Ser Ala Asn Leu Leu Ala Glu Ala Lys Lys Leu Asn Asp Ala
               100                 105                 110
   Gln Ala Pro Lys
           115
<210> 4
   <211> 116
   <212> PRT
   <213> artificial sequence
<220>
   <223> S. aureus Protein A BB domain
<400> 4
   Ala Asp Asn Lys Phe Asn Lys Glu Gln Gln Asn Ala Phe Tyr Glu Ile
   1               5                   10                  15
   Leu His Leu Pro Asn Leu Asn Glu Glu Gln Arg Asn Gly Phe Ile Gln
               20                  25                  30
   Ser Leu Lys Asp Asp Pro Ser Gln Ser Ala Asn Leu Leu Ala Glu Ala
           35                  40                  45
   Lys Lys Leu Asn Asp Ala Gln Ala Pro Lys Ala Asp Asn Lys Phe Asn
       50                  55                  60
   Lys Glu Gln Gln Asn Ala Phe Tyr Glu Ile Leu His Leu Pro Asn Leu
   65                  70                  75                  80
   Asn Glu Glu Gln Arg Asn Gly Phe Ile Gln Ser Leu Lys Asp Asp Pro
                   85                  90                  95
   Ser Gln Ser Ala Asn Leu Leu Ala Glu Ala Lys Lys Leu Asn Asp Ala
               100                 105                 110
   Gln Ala Pro Lys
           115
<210> 5
   <211> 11
   <212> PRT
   <213> artificial sequence
<220>
   <223> HIV-1 Tat 38-48 peptide (core region)
<400> 5
   Phe Thr Lys Lys Gly Leu Gly Ile Ser Tyr Gly
   1               5                   10
<210> 6
   <211> 154
   <212> PRT
   <213> artificial sequence
<220>
   <223> Diphteria Toxin R domain named DTR or DTR-BD
<400> 6
   Pro Gly His Lys Thr Gln Pro Phe Leu His Asp Gly Tyr Ala Val Ser
   1               5                   10                  15
   Trp Asn Thr Val Glu Asp Ser Ile Ile Arg Thr Gly Phe Gln Gly Glu
               20                  25                  30
   Ser Gly His Asp Ile Lys Ile Thr Ala Glu Asn Thr Pro Leu Pro Ile
           35                  40                  45
   Ala Gly Val Leu Leu Pro Thr Ile Pro Gly Lys Leu Asp Val Asn Lys
       50                  55                  60
   Ser Lys Thr His Ile Ser Val Asn Gly Arg Lys Ile Arg Met Arg Cys
   65                  70                  75                  80
   Arg Ala Ile Asp Gly Asp Val Thr Phe Cys Arg Pro Lys Ser Pro Val
                   85                  90                  95
   Tyr Val Gly Asn Gly Val His Ala Asn Leu His Val Ala Phe His Arg
               100                 105                 110
   Ser Ser Ser Glu Lys Ile His Ser Asn Glu Ile Ser Ser Asp Ser Ile
           115                 120                 125
   Gly Val Leu Gly Tyr Gln Lys Thr Val Asp His Thr Lys Val Asn Ser
       130                 135                 140
   Lys Leu Ser Leu Phe Phe Glu Ile Lys Ser
   145                 150
<210> 7
   <211> 21
   <212> PRT
   <213> artificial sequence
<220>
   <223> HIV-1 Tat 37-57 peptide (core and basic regions)
<400> 7
   Cys Phe Thr Lys Lys Gly Leu Gly Ile Ser Tyr Gly Arg Lys Lys Arg
   1               5                   10                  15
   Arg Gln Arg Arg Arg
               20
<210> 8
   <211> 101
   <212> PRT
   <213> artificial sequence
<220>
   <223> HIV-1 Tat consensus sequence
<400> 8
   Met Glu Pro Val Asp Pro Lys Leu Glu Pro Trp Lys His Pro Gly Ser
   1               5                   10                  15
   Gln Pro Lys Thr Ala Cys Asn Asn Cys Tyr Cys Lys Lys Cys Cys Phe
               20                  25                  30
   His Cys Gln Val Cys Phe Thr Lys Lys Gly Leu Gly Ile Ser Tyr Gly
           35                  40                  45
   Arg Lys Lys Arg Arg Gln Arg Arg Arg Ala Pro Gln Asp Ser Gln Thr
       50                  55                  60
   His Gln Val Ser Leu Ser Lys Gln Pro Ala Ser Gln Pro Arg Gly Asp
   65                  70                  75                  80
   Pro Thr Gly Pro Lys Glu Ser Lys Lys Lys Val Glu Arg Glu Thr Glu
                   85                  90                  95
   Thr Asp Pro Val Asp
               100
<210> 9
   <211> 60
   <212> PRT
   <213> Naja nigricollis
<400> 9
   Leu Lys Cys Asn Gln Leu Ile Pro Pro Phe Trp Lys Thr Cys Pro Lys
   1               5                   10                  15
   Gly Lys Asn Leu Cys Tyr Lys Met Thr Met Arg Ala Ala Pro Met Val
               20                  25                  30
   Pro Val Lys Arg Gly Cys Ile Asp Val Cys Pro Lys Ser Ser Leu Leu
           35                  40                  45
   Ile Lys Tyr Met Cys Cys Asn Thr Asp Lys Cys Asn
       50                  55                  60
<210> 10
   <211> 8
   <212> PRT
   <213> artificial sequence
<220>
   <223> ovalbulmine T CD8+ epitope
<400> 10
   Ser Ile Ile Asn Phe Glu Lys Leu
   1               5
<210> 11
   <211> 5
   <212> PRT
   <213> artificial sequence
<220>
   <223> ovalbuline T CD8+ epitope flanking sequence
<400> 11
   Leu Glu Gln Leu Glu
   1               5
<210> 12
   <211> 5
   <212> PRT
   <213> artificial sequence
<220>
   <223> ovalbuline T CD8+ epitope flanking sequence
<400> 12
   Thr Glu Trp Thr Ser
   1               5
<210> 13
   <211> 13
   <212> PRT
   <213> artificial sequence
<220>
   <223> ovalbumine T CD4+ epitope
<400> 13
   Ser Tyr Lys Lys Val Trp Arg Asp His Arg Gly Thr Ile
   1               5                   10
<210> 14
   <211> 11
   <212> PRT
   <213> artificial sequence
<220>
   <223> HIV-1 Tat 47-57 peptide (basic region)
<400> 14
   Tyr Gly Arg Lys Lys Arg Arg Gln Arg Arg Arg
   1               5                   10